# EUROPEAN PATENT SPECIFICATION

(11) **EP 3 912 955 B1**
(45) Date of publication and mention of the grant of the patent: **01.01.2025**
(21) Application number: 20175412.4
(22) Date of filing: 19.05.2020
(51) Int. Cl.: B82Y 15/00, G01N 27/414

(54) **GRAPHENE-INDUCED ENERGY TRANSFER FOR THE QUANTIFICATION OF THE STRUCTURE AND DYNAMICS OF BIOMOLECULES**
GRAPHENINDUZIERTER ENERGIETRANSFER ZUR QUANTIFIZIERUNG DER STRUKTUR UND DYNAMIK VON BIOMOLEKÜLEN
TRANSFERT D'ÉNERGIE INDUIT PAR GRAPHÈNE POUR LA QUANTIFICATION DE LA STRUCTURE ET DE LA DYNAMIQUE DE BIOMOLÉCULES

(43) Date of publication of application: 24.11.2021
(73) Proprietor: Universität Osnabrück, 49074 Osnabrück (DE)
(72) Inventor: PIEHLER, Jacob, 49076 Osnabrück (DE); YOU, Changjiang, 49076 Osnabrück (DE)
(74) Representative: Grünecker Patent- und Rechtsanwälte PartG mbB

(56) References cited:
- WO-A1-2018/013586
- US-A1- 2007 284 557
- HABIBULLA IMRAN ET AL: "Graphene oxide supported liposomes for efficient label free electrochemical DNA biosensing", SENSORS AND ACTUATORS B: CHEMICAL, vol. 260, 9 January 2018 (2018-01-09), NL, pages 841 - 851, XP055736473, ISSN: 0925-4005, DOI: 10.1016/j.snb.2018.01.103
- SHI-WEI CHU: "Optical microscopy approaches angstrom precision, in 3D!", LIGHT: SCIENCE & APPLICATIONS, vol. 8, no. 1, 1 December 2019 (2019-12-01), XP055735159, DOI: 10.1038/s41377-019-0226-y
- SIVA KUMAR KRISHNAN ET AL: "A review on graphene-based nanocomposites for electrochemical and fluorescent biosensors", RSC ADVANCES, vol. 9, no. 16, 18 March 2019 (2019-03-18), pages 8778 - 8881, XP055736520, DOI: 10.1039/C8RA09577A
- GHOSH ARINDAM ET AL: "Graphene-based metal-induced energy transfer for sub-nanometre optical localization", NATURE PHOTONICS, NATURE PUBLISHING GROUP, UK, vol. 13, no. 12, 2 September 2019 (2019-09-02), pages 860 - 865, XP036929062, ISSN: 1749-4885, [retrieved on 20190902], DOI: 10.1038/S41566-019-0510-7
- RICARDO M R ADÃO ET AL: "Graphene setting the stage: tracking DNA hybridization with nanoscale resolution", 2D MATERIALS, vol. 6, no. 4, 30 September 2019 (2019-09-30), pages 045056, XP055736967, DOI: 10.1088/2053-1583/ab41e0
- LARA JORDE ET AL: "Specific and reversible protein immobilization on conductive carbon nanomaterials", CMD2020GEFES, 30 June 2020 (2020-06-30), pages 1, XP055737185, Retrieved from the Internet <URL:https://eventos.uam.es/_files/_event/_28512/papers/48733/v49014/CMD_LaraJorde.pdf> [retrieved on 20201006]

## Description

### FIELD OF THE INVENTION

The present invention relates in general to structure and activity determination of biomolecules. More specifically, the present invention relates to methods of manufacturing devices having a support layer and a biofunctionalized graphene layer on top of the support layer, and the use of such devices for hybridization, determining the structure and structural dynamics of biomolecules, in particular nucleotides, proteins and protein complexes. The present invention also relates to novel compounds for biofunctionalization of graphene, in particular novel amphiphilic molecules for noncovalent surface coating of a graphene layer, and their use in the manufacturing methods and devices of the present invention.

### BACKGROUND OF THE INVENTION

Knowledge on the structure of protein and protein complexes is of key importance for understanding their function, in particular in the context of pharmaceutical applications. Fluorescence methods based on Foerster (Fluorescence) resonance energy transfer (FRET) has been used extensively for exploring protein structures. Without demanding instrumentation, FRET and its derivatives, luminescence (or lanthanide-based) resonance energy transfer (LRET), report structural dynamics of proteins in complex biological milieu of live cells.

The rate of FRET decays dramatically with a (r/R₀)⁻⁶ dependency of the intermolecular distance *r* and Foerster radius R₀. Since R₀ is typically a few nanometers, the effective range of FRET is within 10 nm. However, many proteins possess a hydrodynamic radius extending above this distance. It is also quite common that protein complexes composed of multiple subunits have large dimension in tens of nanometers. Therefore, there is an essential need in the art to develop methods with spatial accuracy similar to FRET but extended sensitivity distance.

Fluorescence quenching of fluorophores nearby to graphene has been implemented for a broad application in biosensing. In case of the excited fluorophores near graphene but without contact, they undergo long distance radiationless electromagnetic coupling into graphene plasmon. This phenomenon is similar to metal-induced energy transfer (MIET) (Chizhik et al. 2014, Nature Photonics, Vol. 8, 124) and is hereafter termed as graphene induced energy transfer (GIET).

The atomic thickness of graphene ensures high optical transparency and an extremely high confinement of the plasmon. Theoretical calculations have predicted that the energy transfer rate of GIET has a *d*⁻⁴-dependency on the vertical distance *d* to graphene (Swathi et al. 2009, The Journal of Chemical Physics, Vol. 130(8), 086101). The sensitive region of GIET is predicted up to 30 nm, which is very appealing for structural biology. However, for applications of GIET in protein structure analysis, surface biofunctionalization of graphene is a crucial prerequisite. Deterioration of graphene electronic properties must be avoided, while the structural integrity of protein and protein complexes shall be maintained. Commonly used graphene oxidation introduces modifications on the carbon bond. These modifications may affect the graphene plasmon (Dreyer et al. 2010, Chemical Society Reviews, Vol. 39(1), 228), thereby changing the distance dependent manner of GIET. For these reasons, noncovalent biofunctionalization of graphene would be desirable. Furthermore, site-specific immobilization of proteins on non-covalently modified graphene would be desirable to obtain intact protein functionality and controlled protein orientation. WO201813576 shows a graphene sensor device, that comprises support substrate, graphene layer, functionalization layer (on top of graphene layer) and electrodes. The device comprises a lipid bilayer, on the functionalization layer.

There is a need in the art for novel methods of using GIET for quantitatively detecting structure and dynamics of a large spectrum of biomolecules and developing new compounds for non-covalent biofunctionalization of graphene. Such GIET based methods and products thereof can reduce sample consumption and save time in analysis, while an automatic large scale screening of drug candidates can be realized by integrating GIET devices within a fluidic system, sampling system, etc. The present invention addresses the need in the art by the subject matter disclosed and claimed herein.

### SUMMARY OF THE INVENTION

The present invention provides a novel method of manufacturing devices having a support layer and a biofunctionalized graphene layer on top of the support layer, and the use of such devices for hybridization, determining the structure and dynamics of biomolecules, in particular nucleotides, proteins and protein complexes. Furthermore, the present invention provides novel compounds for biofunctionalization of graphene, in particular novel amphiphilic molecules for noncovalent surface coating of a graphene layer, and their use in the manufacturing methods and devices of the present invention.

It has surprisingly been found that the distance dependency of graphene induced energy transfer (GIET) can be calibrated and thereby used for high precision quantitative analysis of size and dynamics of structurally defined biomolecules, including, e.g., nucleotides, protein, protein complex, protein with an intrinsically disordered domain, or protein complexes with dimensions of up to 50 nm. The present invention provides biofunctionalization of graphene layers for site-specific immobilization of biomolecules for determination of structure or conformational changes. It has surprisingly been found that using the non-covalent biofunctionalization of graphene of the present invention in GIET based methods, the structure of biomolecules remains intact and their orientation can be controlled. Thus, the function of biomolecules with respect to the interactions with other molecules is preserved.

GIET based methods of the present invention may be characterized as including three main steps:
(i) transfer of a (commercially available) graphene (mono)layer onto a support layer, e.g., a glass substrate;
(ii) surface biofunctionalization of the graphene layer for site specific immobilization of biomolecules; and
(iii) determination of fluorescence quenching to obtain the vertical distance of fluorophores to graphene.

In one aspect, the present invention provides a method of manufacturing a device comprising a layer of bio-functionalized graphene on top of a support layer. In a second aspect, the present invention provides new amphiphilic molecules for non-covalent surface coating of graphene layers. The hydrophobic terminal may exploit van der Waals interaction by lipid analogue, or pi-pi interaction via pyrene or dibenzocyclooctyne (DBCO). An affinity capturing moiety, such as tris-nitrilotriacetic acid (tris-NTA), may be introduced to the other terminal for site-specific, reversible immobilization of biomolecules. The termini may be connected by polyethylene glycol (PEG). In a third aspect, the present invention provides devices manufactured by the methods disclosed herein. In a fourth aspect, the present invention provides the use of a manufactured device disclosed herein for validation of distance dependent GIET by fluorescence intensity and/or fluorescence lifetime. The transparency of graphene allows lifetime determination assays completed very quickly. DNA may be used as geometrically defined nanorulers. In a fifth aspect of the invention, means and methods disclosed herein for biofunctionalized graphene on a support layer can be used as device in particular for DNA hybridization assays that are involved in clinical PCR (polymerase chain reaction) tests of target genomic sequences. In a sixth aspect, the present invention provides methods of using the manufactured devices disclosed herein for determining the size, structure or dynamics including conformational changes of biomolecules, in particular of nucleotides, structurally defined proteins, protein complexes, proteins with an intrinsically disordered domains, or protein complexes with dimensions of up to 50 nm. In a seventh aspect of the invention, the methods, devices, and compositions provided herein can be used in analytical or diagnostic applications.

The present invention is defined in the claims.

### BRIEF DESCRIPTION OF THE DRAWINGS

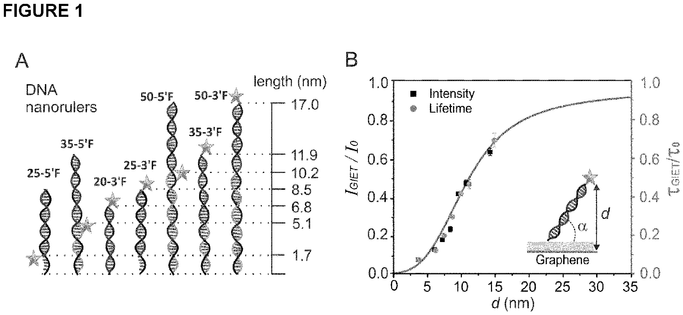

**Figure 1****: Calibration of distance-dependent GIET. (A)** Scheme of the DNA nanorulers. Probe strand is conjugated with fluorescein (shown as stars) at either 3'- or 5'-end, respectively. **(B)** Plots of the unquenched fluorescence measured by intensity ratio (squares, left axis) and lifetime ratio (dots, right axis) as a function of the vertical distance d from the fluorophore to graphene surface. Value is mean ± sd from 3 independent measurements. Curve shows the theoretical prediction. Inset is the scheme for calculating d. Gray area on top of graphene indicates the noncovalent coating on graphene.
**Figure 2****: Hexahistidine-tagged antiGFP nanobody and GFP binding detected by surface sensitive RIfs-TIRFs. (A)** Mass signal detected by reflectance interference spectroscopy (RIfs) for lipid coating (I), His-tagged NB immobilization (III), and tag-less mEGFP binding (IV), marked by gray bars. **(B)** Simultaneous time-lapse fluorescence intensity traces. Dash: silica transducer. Line: graphene-coated silica transducer.
**Figure 3****: Axial distance of NB::GFP complex and GFP determined by GIET. (A)** IGIET/I0 ratios of NB::GFP complex and GFP obtained by surface sensitive Rlfs-TIRFs measurements. **(B)** Cartoons showing the quantified axial distance.
**Figure 4****: Quantification of conformational change of DNA by GIET. (A)** Schematic illustration of the 35 mer ssDNA anchor strand's conformational changes on graphene. **(B)** Time correlated single photon counting of probe strand hybridized with the 35 mer anchor DNA strand at different conditions. Gray curve: in absence of 15 mer blocker on graphene. Black curve 2: in the presence of blocker on graphene. Black curve 1: in the presence of blocker on glass.
**Figure 5****: Molecular structure of trisNTA-PEG-DBCO and trisNTA-PEG-Pyrene.**
**Figure 6****: Label-free solid phase detection of trisNTA-PEG-Pyrenel PEG-Pyrene coating.** (I), conditioning and Ni²⁺ loading (II), and immobilization of hexahistidine-GFP (III). (IV) is imidazole wash step.
**Figure 7****: Label-free solid phase detection of GFP binding on HaloTag-DARPin::BSA-HTL-functionalized graphene** (Black line). Grey line shows negative control with negligible GFP binding on BSA-functionalized graphene.

### DETAILED DESCRIPTION OF THE INVENTION

The present invention provides a method of manufacturing a device having a support layer and a biofunctionalized graphene layer on top of the support layer, characterized in that the method comprises the steps:
(a) transferring a layer of graphene to a support layer to form a support layer with a graphene layer on top;
(b) applying an amphiphilic coating to the graphene layer of step (a) to form a non-covalent surface coating on the graphene layer; and
(c) obtaining a device having a support layer and a biofunctionalized graphene layer on top of the support layer.

The term 'device', as used herein, denotes any instrumentation suitable for assaying samples by means of the method and may be used interchangeably with the term 'chip'. Typical devices of the invention are manufactured according to the methods described herein. According to the invention, the term 'device' refers to an object for quantitative measurement and/or detection of the structure and dynamics of a biomolecule. In the present invention, the device comprises a layer of bio-functionalized graphene on top of a support layer. The device may comprise a solid surface on top of the support layer. The term 'solid surface' refers to a material having a rigid or semi-rigid surface. As used herein, a device is designed to be contacted with a sample having one or more biomolecules which may or may not have the capability of binding to the (support layer or solid surface of the) device. The solid surface can be chosen for its intrinsic ability to attract and immobilize biomolecules. Alternatively, the solid surface can retain an attachment molecule which has the ability to attract and immobilize the biomolecule. As described herein, the bio-functionalized graphene layer may be on top of a solid surface of the support layer.

The term 'graphene', as used herein, incorporates the ordinary meaning of the term and describes pure or nearly pure carbon in a one-atom thick layer where carbon atoms are densely packed in a regular sp2-bonded atomic-scale hexagonal pattern. 'Graphene layer' or a 'layer of graphene' as used herein encompasses not only a graphene monolayer, but may also encompass a plurality of layers of graphene. For convenience, a singular 'graphene layer' or 'a layer of graphene' is used throughout the disclosure which may refer to a single layer similar to a graphene monolayer or a plurality of graphene layers. In various embodiments, the graphene layer may comprise a plurality of graphene layers, including two, three, four, five etc. graphene layers. In preferred embodiments, a graphene layer is a graphene monolayer. Furthermore, 'graphene layer' or a 'layer of graphene' as used herein encompasses not only a continuous graphene layer but also non-continuous graphene layer(s). In a preferred embodiment, the graphene layer is continuous. The term 'continuous' as used herein means a coating of the graphene layer having an uninterrupted extension along a surface. Other ways to describe continuously include but are not limited to 'constantly' or 'reoccurring in rapid succession'. Accordingly, as used herein, the term 'continuous' refers to a layer that covers an entire surface without gaps or bare spots that reveal material underlying the layer, i.e. the support layer. The term 'continuous' as used herein is meant to encompass the graphene layer having unintentional, intermittent, isolated interruptions or gaps (e.g., from a manufacturing defect) from a discontinuous deposit. These gaps or bare spots on the surface may be less than about 1 % of the total surface area. Whereas in embodiments where the graphene layer is non-continuous, there is no particular requirement for the spatial arrangement of the non-continuous graphene layer. They can be arranged periodically or non-periodically on the support layer. In all of the above embodiments, there are no preferred shapes of the graphene layer, which include but are not limited to squares, stripes, spheres, triangles, ellipse, or other irregular shapes. In various embodiments, the graphene layer is at least 100 nm in length. In a preferred embodiment, the graphene layer is larger than 100 nm in length. Other preferred length scales of the graphene layer are at least 200 nm, 300 nm, 400 nm, 500 nm, 600 nm, 700 nm, 800 nm, 1 µm. Even more preferably larger than 1 µm, 10 µm, 100 µm and 1 mm, 5 mm. In various embodiments, the thickness of the graphene monolayer used according to the invention is approximately 0.34 nm. In other embodiments, the thickness is up to 0.5 nm, preferably up to a few nanometers depending on the number of graphene layers.

The term 'support layer', as used herein, refers to the layer that provides a mechanical support for the layer of bio-functionalized graphene. As used herein, support layer is a material having a rigid or semi-rigid surface. In various embodiments, such as in the case of the support layer upon which a layer of bio-functionalized graphene is formed, at least one surface of the support layer will be substantially flat. In the case of the compound or material library, it may be desirable to physically separate synthesis regions in the support layer for different materials with, for example, dimples, wells, raised regions, etched trenches, or the like. In some embodiments, the support layer for the compound or material library will itself contain wells, raised regions, etched trenches, etc. which form all or part of the synthesis regions. In one embodiment of the present invention, the support layer provides a suitably strong foundation to which the layer of bio-functionalized graphene is transferred. In a preferred embodiment, the support layer of the present invention is a silica-based support layer. 'Silica' as used herein, is a material that has silicon (Si) and oxygen (O) atoms, and possibly additional substituents such as, but not limited to, other elements such as H, C, B, P, or halide atoms; alkyl groups; or aryl groups. In certain instances, the material may further comprise silicon-carbon bonds having a total number of Si-C bonds relative to the total number of Si atoms ranging from between about 20 to about 80 mole percent, or from between about 40 to about 60 mole percent. Preferably, the silica support layer comprises any one of a silica-type material, a UV-curable plastic material, glass, silicon, and/or a thermal plastic material, preferably the support layer comprises a silica-type substrate, more preferably silica glass, which may be selected from the group consisting of 1x1 cm² silica-coated transducers or glass microscopy coverslips.

In preferred embodiments, silica as used herein is in the form of silica-coated transducers. The term 'transducer' as used herein refers to any kind of functional component or an arrangement of functional components configured to convert energy from one form to another and/or configured to convert an input signal. In a further preferred embodiment, silica-coated transducers are for the purpose of reflectance interference spectroscopy and total internal reflection fluorescence spectroscopy detection (Rlfs-TIRFs). In another preferred embodiment, silica substrates as used herein are glass microscopy coverslips. 'Coverslip,' as used herein, refers to a thin slip of glass for supporting a biological specimen that to be observed under a microscope. The coverslip should be of a sufficient length and width to support the biological specimen in its entirety.

In various embodiments of the invention, the manufacturing method disclosed herein comprises contacting the graphene layer with the support layer. In one embodiment, the graphene layer comprises a protective polymer layer. As described herein, 'transferring' or 'contacting' refers to face-to-face contact in order to coat the support layer with a layer of graphene. In other embodiments, the manufacturing method involves drying the support layer with the graphene layer, e.g., at room temperature (e.g., at 25°C). The obtained support layer with the graphene layer is preferably dried at room temperature in a time controlled manner, preferably between 15 minutes to 45 minutes, more preferably for about any of 20 minutes, 25 minutes, 30 minutes, 35 minutes, 40 minutes or 45 minutes. In various preferred embodiments, the support layer with the graphene layer is dried at room temperature for 30 minutes. As used herein, room temperature may encompass any temperature between 20°C and 25°C, preferably room temperature means about 25°C. Following the step of drying at room temperature, the support layer with the graphene layer may be heated at a temperature between 100°C to 200°C, including, e.g., at about any of 100°C, 120°C, 150°C, 170°C, 180°C, and 200°C. In a preferred embodiment, it is heated at a temperature of about 150°C. The step of heating may be performed for an interval between 1 to 2 hours, for 1 hour, 1.5 hours, and most preferably for 2 hours. The heated product may be stored under vacuum for cooling down, preferably for 24 hours. In various embodiments, the cooled product is then incubated in two to three organic solvents. In preferred embodiments, the cooled product may be incubated in two organic solvents in order to remove the protection polymer layer from the graphene layer. In further preferred embodiments, the incubation may be done sequentially in two organic solvents. In other preferred embodiments, the cooled product may be first incubated in acetone, preferably for 1 hour, and then in isopropanol for another 1 hour. In further embodiments, the obtained support layer with graphene monolayer may be blow dried with Nitrogen (N₂). In all of the above embodiments, the support layer is preferably a silica-based support layer. Preferably, the silica-based support layer comprises any one of a silica-type material, a UV-curable plastic material, glass, silicon, and/or a thermal plastic material, preferably the support layer comprises a silica-type substrate, more preferably silica glass, which may be selected from the group consisting of 1x1 cm² silica-coated transducers or glass microscopy coverslips.

A person skilled in the art is aware that transferring of graphene to the support layer can be obtained by other methods. In one embodiment, the graphene for transferring may have a copper foil as substrate which could be removed by etching solutions during the transferring process. The etching solution contains any one of ammonium persulfate, aluminum chloride, ferric chloride, ferric nitrate, or their combinations thereof. In a further embodiment, the direct synthesis process may be used to 'transfer' graphene layers on the support. The graphene layer is formed on the support layer by magnetron sputtering, electron beam evaporation, or chemical vapor deposition using a gas or solid carbon source. The preferred gas carbon source is any one of methane/hydrogen, ethylene/hydrogen, or acetylene/hydrogen. The preferred solid carbon source is any one of amorphous carbon, graphite, fullerene, carbon nanotube, or the combinations thereof. In various embodiments, the graphene may be not flawless during the manufacturing process. The obtained graphene layer may contain oxygen, hydrogen, amine group, carboxyl group, or metal ions. In these embodiments, unless the content of non-carbon elements makes graphene unable to be modified with the amphiphilic molecules, those graphene layers can be treated as the flawless graphene layer.

The method of manufacturing a device as described herein comprising a layer of biofunctionalized graphene on top of a support layer according to the first aspect of the invention also comprises functionalization of the graphene layer with synthesized compounds of the invention disclosed herein. Functionalization of graphene as used herein refers to reacting graphene with a functional group precursor to form a functionalized graphene layer. The term 'biofunctionalization' of graphene as used herein refers to the formation of a graphene layer for modulating the interaction and/or immobilization of biomolecules to the graphene monolayer. Accordingly, in some embodiments, provided herein is a method of producing a graphene layer with tailored surface properties by (bio)functionalization. Methods described herein may advantageously be performed without the need for harsh oxidation and/or reduction of graphene in order to attach functional groups, which can introduce many hole defects on the graphene sheets or other defects destroying the pi electron cloud on graphene, reducing its conductivity and thus performance. Additionally, methods for synthesizing functionalized graphene layers may be simplified, scalable, and cost-effective, without need for long reaction times, harsh power sonication, surfactants, reactive graphite intercalation compounds, and/or non-ideal electrochemical methods. Using the embodiments described herein, graphene layers including a wide variety of functional groups may be synthesized in high yield. Such molecules may be utilized alone or with nanoparticles or polymers. In preferred embodiments, the functionalized layer of graphene may be a single-layer (bio)functionalized graphene . In some cases a (bio)functionalized layer of graphene may be a multi-layer (bio)functionalized graphene. The functional group precursor may be any species capable of forming a bond with graphene. The bond formation of the functional group with graphene may be covalent or non-covalent, thereby referring to covalent and/or non-covalent surface coating. In the manufacturing methods of the present invention, the (bio)functionalization is a non-covalent surface coating. As used herein, 'non-covalent' (bio)functionalization of graphene may be based on van der Waals forces, or π-π interactions with any of the compounds disclosed herein for (bio)functionalization of the graphene layer. The non-covalent interactions are particularly suitable for applications of the present invention because the extended π system of graphene nanostructures has been proven not to be interrupted, which means that properties such as graphene plasmon, electric conductivity or mechanical strength are not affected. The non-covalent (bio)functionalization of graphene with amphiphilic biofunctionalization compounds disclosed herein does not interrupt the electrical or mechanical properties of the graphene layer. Moreover, the non-covalent (bio)functionalization of graphene is obtained by direct contact of the amphiphilic biofunctionalization compounds with the graphene layer. The thickness of the (bio)functionalization layer on top of graphene according to the invention is approximately at least 0.3 nm, between 0.3 to 0.5 nm, between 0.5 to 0.7 nm, between 0.7 to 0.8 nm, between 0.8 to 0.9 nm, between 0.9 to 1.0 nm or at least 1.0 nm. In some embodiments, the thickness of the (bio) functionalization layer on top of graphene according to the invention is approximately up to 0.3 nm, 0.5 nm, 0.7 nm, 0.8 nm, 0.9 nm, 1.0 nm, 1.5 nm, 2.0 nm, 2.5 nm, 3.0 nm, 3.5 nm, 4.0 nm, 4.5 nm, or up to 5.0 nm. The thickness of the (bio) functionalization layer on top of graphene serves as the lower detection limit of the GIET device of the invention. Therefore, the modulation of the interaction and/or immobilization of biomolecules on graphene layer can be achieved with an advantageous minimum distance above graphene. Said minimum distance, as described above, depends on the thickness of the (bio) functionalization layer and is 0.3 nm, 0.5 nm, 0.7 nm, 0.8 nm, 0.9 nm, 1 nm, 1.5 nm, 2 nm, 2.5 nm, 3 nm, 3.5 nm, 4 nm, 4.5 nm, or 5 nm. Accordingly, the sensitive GIET region of the present invention can have a lower limitation as the minimum distance.

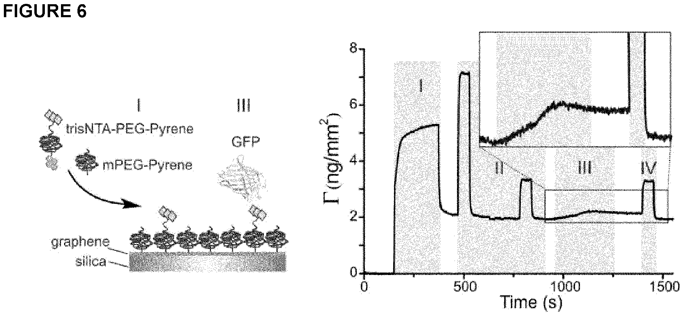

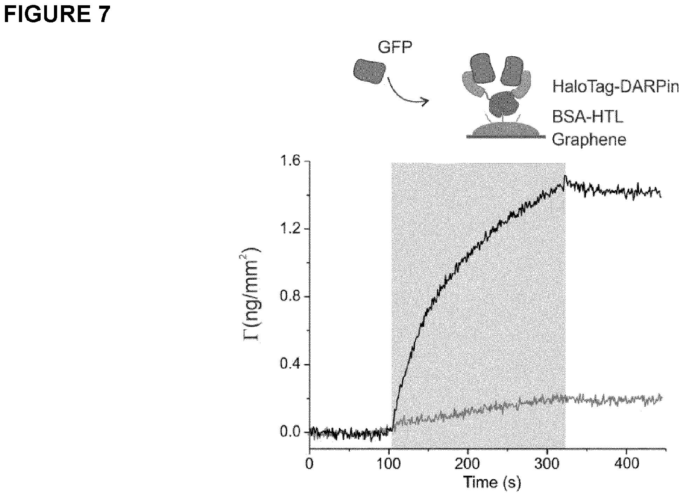

In exemplary embodiments, methods of (bio)functionalization of the graphene layer with amphiphilic biofunctionalization compounds of the invention include the use of trisNTA-PEG-Pyrene and BSA-HaloTag Ligand conjugate, which are also provided by the present invention. Formation of a (bio)functionalization layer of said amphiphilic compounds on a graphene layer may be characterized by label-free solid phase detection as shown in Figure 6 for trisNTA-PEG-Pyrene, and Figure 7 for BSA-HaloTag Ligand conjugate. In preferred embodiments, the non-covalent interaction between the amphiphilic compounds and the graphene layer is via the hydrophobic terminal of the compounds. In various embodiments, the formation of the functionalization layer on graphene is characterized by a mass signal between any one of 0.2 to 10.0 ng/mm², 0.2 to 1.0 ng/mm², 1.0 to 2.0 ng/mm², 2.0 to 2.5 ng/mm², 2.5 to 3.0 ng/mm², 3.0 to 3.5 ng/mm², 3.5 to 4.0 ng/mm², 4.0 to 4.5 ng/mm², 4.5 to 5.0 ng/mm², 5.0 to 6.0 ng/mm², 6.0 to 8.0 ng/mm², and 8.0 to 10.0 ng/mm². A mass signal change by 1 ng/mm² corresponds to a shift of the interference minimum (1.5^{th} order) by 1.2 nm as determined by calibration experiments. The mass signal confirms stable pi-pi interaction between the amphiphilic biofunctionalization compounds and the graphene layer. All of the above mentioned embodiments of the invention lead to obtaining a device having a support layer and a biofunctionalized graphene layer on top of the support layer.

The manufacturing method of the present invention encompasses a step of applying an amphiphilic coating to the graphene layer to form a non-covalent surface coating on the graphene layer. Furthermore, according to the second aspect, the present invention also provides newly synthesized (amphiphilic) compounds for the biofunctionalization of graphene on top of a support layer. As described herein, the amphiphilic coating comprises amphiphilic molecules having a hydrophobic group and a hydrophilic group that may or may not be connected by a linker.

The term 'amphiphilic molecules' as used herein generally refers to molecules having both hydrophilic and hydrophobic parts that can self-assemble in water or another solvent to form structures such as vesicles or micelles. As used herein, the term 'hydrophilic group' means a polar group having a strong affinity for water, and the term 'hydrophobic group' means a nonpolar group, having a low affinity for water and a high affinity for oil. The definition of the hydrophobic group and the hydrophobic group in the present invention may include all hydrophilic groups and hydrophobic groups, which are widely known to those skilled in the art. According to the invention, amphiphilic molecules are used to coat the graphene layer on top of the support layer known as (bio)functionalization as described elsewhere herein. In such embodiments, amphiphilic molecules are also known as matrix molecules.

In various embodiments, the hydrophobic terminal is bound to the graphene layer. In some embodiments, the hydrophobic group bound to the graphene layer is a polycyclic aromatic hydrocarbon derivative. The polycyclic aromatic hydrocarbons referred to herein comprise hydrocarbons composed of multiple aromatic rings that are fused, i.e. share one or more sides. Polycyclic aromatic hydrocarbons are also known as polynuclear aromatics ('PNA'). Thus, the terms 'polycyclic aromatic hydrocarbons', 'PCA hydrocarbons', 'PCAs', 'polynuclear aromatic compounds' or 'PNAs' may be used interchangeably herein to refer to aromatic hydrocarbons having multiple fused aromatic rings. In preferred embodiments, the polycyclic aromatic hydrocarbon as used herein is pyrene. The term 'pyrene' is known to those skilled in the art. It refers to a polycyclic aromatic hydrocarbon (PAH) consisting of four fused benzene rings, resulting in a flat aromatic system. The chemical formula is C₁₆H₁₀. The general structure of Pyrene is given by:

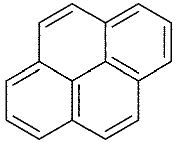

In various embodiments, the hydrophobic group bound to the graphene layer is a cycloalkyne. The term 'cycloalkyne', as used herein, refers to an unsaturated monocyclic hydrocarbon group having at least one endocyclic triple bond. Examples of cycloalkynes include, but are not limited to, cyclooctyne and cyclononyne. In a preferred embodiment, the cycloalkyne as used here is a cyclooctyne derivative. 'Cyclooctyne' as used herein refers to an 8-membered ring containing a carbon-carbon triple bond. In a further preferred embodiment, cyclooctyne derivative as used here is a benzo-fused cyclooctyne derivative. In more preferred embodiments, the benzo-fused cyclooctyne derivative is a dibenzocyclooctyne (DBCO). The term 'DBCO' is known to those skilled in the art. It refers to a dibenzo fused cycloalkyne compound composed of an 8-membered ring. The chemical formula is C₁₆H₁₂. The general structure of DBCO is given by:

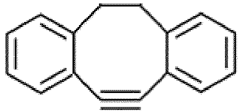

In some embodiments, the hydrophobic group bound to the graphene layer is an aliphatic amine. The term 'aliphatic amine', as used herein, refers to an amine compound in which the nitrogen atom of an amino group is connected to an aliphatic group, not an aromatic ring, for example, , butylamine, , cyclohexylamine, cyclohexane-diamines, or the like. The term 'aliphatic diamine', as used herein, refers to a subset of aliphatic amine compounds which contain two amino groups. In a preferred embodiment, the aliphatic amine as used herein is a fatty amine derivative. The term fatty amine as used throughout the description is meant to denote an aliphatic amine with at least one fatty alkyl chain, with a fatty alkyl chain being a saturated or unsaturated carbon. The fatty amine may comprise more than one amine moiety. Suitable fatty amines are primary, secondary or tertiary fatty amines such as n-decyl amine, n-dodecyl amine, (coco alkyl)amine, n-tetradecyl amine, n-hexadecyl amine, n-octadecyl amine, oleyl amine, (tallow alkyl)amine, (rapeseed alkyl)amine, (soya alkyl)amine, erucyl amine, (coco alkyl)amine, N-(n-decyl)-trimethylene diamine, N-(n-dodecyl)-trimethylene diamine, N-(coco alkyl)-trimethylene diamine, N-(oleyl alkyl)-trimethylene diamine, N-(rapeseed alkyl)-trimethylene diamine, N-(soya alkyl)-trimethylene diamine, N-(tallow alkyl)-trimethylene diamine, N-erucyl trimethylene diamine, N-(n-decyl)-N'-(3-aminopropyl)-1,3-propane diamine, N-(n-dodecyl)-N'-(3-aminopropyl)-1,3-propane diamine, oleyl-1, 3-diaminopropane, N-(coco alkyl)-N'-(3-aminopropyl)-1,3-propane diamine, N-(rapeseed alkyl)-N'-(3-aminopropyl)-1,3-propane diamine, N-(soya alkyl)-N'-(3-aminopropyl)-1,3-propane diamine, N-oleyl-N'-(3-aminopropyl)-1,3-propane diamine, N-(tallow alkyl)-N'-(3-aminopropyl)-1,3-propane diamine, N-erucyl-N'-(3-aminopropyl)-1,3-propane diamine, N-(3-aminopropyl)-N'-[3-(9-decylamino)propyl]-1,3-propane diamine, N-(3-amino-propyl)-N'-[3-(9-dodecylamino)propyl]-1,3-propane diamine, N-(3-aminopropyl)-N'-[3-(9-(coco alkyl)amino)propyl]-1,3-propane diamine, N-(3-aminopropyl)-N'-[3-(9-(rapeseed alkyl)amino)propyl]-1,3-propane diamine, N-(3-aminopropyl)-N'-[3-(9-(soya alkyl)amino) propyl]-1,3-propane diamine, N-(3-aminopropyl)-N'-[3-(9-octadecenylamino)propyl]-1,3-propane diamine, N-(3-aminopropyl)-N'-[3-(9-(tallow alkyl)amino)propyl]-1,3-propane diamine, and N-(3-aminopropyl)-N'-[3-(9-erucylamino)propyl]-1,3-propane diamine. In further preferred embodiments, the fatty amine derivative is an octadecylamine derivative. In particularly preferred embodiments, the octadecylamine derivative is a dioctadecylamine (DODA). The term 'DODA' is known to those skilled in the art. It refers to a secondary amine, and a fatty amine derivative. The chemical formula is [CH₃(CH₂)₁₇]₂NH.The general structure of DODA is given by:

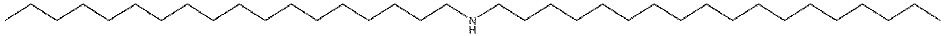

In various embodiments, the hydrophobic group bound to the graphene layer is a surface adhesive protein. The term 'surface adhesive protein' as used herein refers to a blocking agent to prevent non-specific binding of the analyte of interest. To eliminate the residual binding capacity of the surface on which the reaction is carried out, protein-based blocking agents are commonly used. Blocking agents can also stabilize the biomolecules bound to the surface and reduce non-specific interactions. The surface adhesive protein can be any one of steptavidin, neutravidin, avidin, extracellular matrix protein or serum protein. In one embodiment, the extracellular matrix protein is fibronectin, laminin and collagen. The serum protein is one of albumin, macroglobulin, and haptogobin. In another embodiment, antibody can be used as protein adhesive protein on graphene. The antibody is commonly used immunoglobulin comprising heavy chain and light chains. It may also be single chain antibody and nanobody as known by a person skilled in the art. In preferred embodiments, the surface adhesive protein used herein is bovine serum albumin (BSA). The term 'BSA' as used herein is known to those skilled in the art. BSA is a serum albumin protein derived from cows. The full-length BSA precursor protein is 607 amino acids (AAs) in length. An N-terminal 18-residue signal peptide is cut off from the precursor protein upon secretion, hence the initial protein product contains 589 amino acid residues. An additional six amino acids are cleaved to yield the mature BSA protein that contains 583 amino acids.

Accordingly, in particularly preferred embodiments, the hydrophobic group of the amphiphilic compounds of the invention bound to the graphene layer is pyrene, or dibenzocyclooctyne (DBCO), or dioctadecylamine (DODA), or bovine serum albumin (BSA).

In various embodiments, the hydrophilic group of the amphiphilic compounds of the invention may comprise an affinity capturing moiety. As used herein, the term 'affinity' refers to the equilibrium constant for the reversible binding of two agents and is expressed as K_{D}. Affinity of a binding protein to a ligand such as affinity of an antibody for an epitope can be, for example, from about 1000 nanomolar (nM) to about 0.1 nM, from about 100 nM to about 1 picomolar (pM), or from about 100 nM to about 1 femtomolar (fM). 'Affinity-capture' as used herein refers to the capture of target analytes using a moiety specific for a target analyte. As used herein the term 'affinity capturing moiety' refers to a molecule that binds with an affinity moiety-binding partner (e.g. an epitope, a receptor, a ligand etc.) to form an affinity binding pair. Likewise, the term 'affinity moiety-binding partner' refers to a moiety or molecule that binds with an affinity moiety and is used interchangeably with the term 'functional moiety'. The affinity moiety can be synthetic, semi-synthetic, or naturally occurring. The binding can occur through non-covalent interactions, such as electrostatic interaction, dipole-dipole interactions (e.g. Keesom interaction, London dispersion, and Debye interaction), dipole-π interaction, hydrogen bonds, van der Waals contacts, van der Waals/London dispersions, π-π stacking and ionic bonds (e.g., salt bridges). The binding can occur through covalent interactions, leading to formation of, for example, ether bonds, ester bonds, amide bonds, phosphate bonds, schiff base, disulfide bonds, and thiol ester bonds. Illustrative affinity binding pairs irrespective of the target analyte include, for example, avidin or streptavidin and biotin; ligands and receptors; protein A or G binding and Fc-region of immunoglobulin; oligonucleotides and complementary sequences, e.g., polydesoxyadenylic acid and polydesoxythimidylic acid, or polydesoxy-guanylic acid and polydesoxycytidylic acid; Ni-NTA (nitrilotriacetic acid, nickel salt) and poly histidine-tagged ligand, and the like. In some embodiments, the 'affinity capturing moiety' and 'affinity moiety-binding partner' are members of a specific binding pair. A specific binding pair comprises two different moieties/molecules that specifically bind to each other through chemical or physical means. Specific binding partners include antigens/epitopes and their antigen-binding molecules (e.g., antibodies), enzymes and their binding partners (including cofactors, inhibitors and chemical moieties whose reaction the enzymes promote), ligands (e.g., hormones, cytokines, growth factors, vitamins etc.) and their receptors, complementary peptides, specific carbohydrate groups and lectins, antibiotics and their antibodies and naturally occurring binding proteins, complementary nucleotide sequences, aptamers and their targets, biotin and avidin (or streptavidin), and the like. Furthermore, specific binding pairs can include members that are analogs of the original specific binding members, for example, an analyte-analog. Immuno-interactive specific binding members include antigens, antigen fragments and their epitopes, and antigen binding molecules such as but not limited to antibodies, antibody fragments, and variants (including fragments of variants) thereof, whether isolated or recombinantly produced.

In the present invention, the term target analyte capturing moiety refers to any of the affinity capturing moieties described herein pertaining to a specific target analyte, e.g., protein capturing moiety, nucleotide capturing moiety, chelating agents etc.

The term nucleic acid affinity capturing moiety according to the invention includes any of the affinity capturing moiety described herein, e.g., including but not limited to binding proteins, complementary nucleotide sequences, aptamers and their targets, biotin and avidin (or streptavidin), and the like. Each alternative represents a separate embodiment of the invention.In the present invention, the protein affinity capturing moiety may be an enzymatic protein capturing ligand, such as an enzymatic protein capturing ligand of *O*⁶-benzylguanine derivatives or *O*²-benzylcytosine derivatives; preferably the enzymatic protein capturing ligand may be a haloalkane derivative, more preferably a chloroalkane derivative; or a chelating agent, preferably a carboxylic acid-based chelator, more preferably a di- or triacetic acid-based chelator.

In one embodiment, the affinity capturing moiety is *O*⁶-benzylguanine derivative, i.e. the SNAP tag ligand. SNAP-tag is a 182 residues polypeptide (19.4 kDa) that can be specifically and covalently tagged with the SNAP tag ligand. In particularly preferred embodiments, the affinity capturing moiety is a HaloTag Ligand. In further preferred embodiments, the enzymatic protein capturing ligand may be a 6-cholrohexanol derivative, preferably chlorohexane-OEG2-amine. HaloTag as used herein is a self-labeling protein tag that can be fused to protein of interest thus serves as a functional moiety. It may be a 297 residue peptide (33 kDa) derived from a bacterial enzyme, designed to covalently bind to a synthetic ligand, the HaloTag ligand, which can be fused to a protein of interest via the functional moiety.

In various embodiments, the 'affinity capturing moiety' may be a chelating agent. As used herein, the term 'chelating agent' is a chemical compound that coordinates with a metal to form a chelate. It comprises molecules containing two or more electron donor atoms that can form coordinate bonds to a single metal ion. The term 'chelating agent' is understood to include the chelating agent as well as salts thereof. In a preferred embodiment, the chelating agent as used herein is a carboxylic acid-based chelator, more preferably a di- or triacetic acid-based chelator. In one embodiment, the diacetic acid-based chelator is iminodiacetica acid (IDA) which may or may not be preloaded with zinc ions. In the preferred embodiment, the chelating agent as used herein is a nitrilotriacetic acid (NTA) based agent that may be or may not be preloaded with metal ions. The metal ions is any one of nickel ions, zinc ions, cobalt ions, copper ions. An illustrative affinity binding pair of NTA i.e. the functional moiety according to the invention is the poly histidine tag. The term 'poly histidine tag', as used herein, refers to a linear sequence of histidine residues allowing for affinity based immobilization of the peptide and/or protein A poly-His tag may comprise any one of 6, 7, 8, 9,10, 11, 12, 13, 14 consecutive Histidine residues. In various embodiments, the chelating agent is a multivalent chelator.

As further described herein, the hydrophobic group and the hydrophilic group may be connected by a linker, preferably the linker is a polymer, more preferably the linker is poly(ethylene glycole) (PEG).

The term linker as used herein means a moiety that links together at least two other moieties, such as a hydrophobic group and a hydrophilic group in an amphiphilic molecule. A wide variety of linkers may be used, including biodegradable linkers and biopolymers. The linker described herein specifically comprises polymers, such as macromolecular structures, preferably poly (ethylene glycols), referred to as PEGs. The linker as used herein preferably comprises repeat units of PEG. The repeat units are preferably in the range 50-500, at least 400, preferably at least 200, more preferably at least 100, and even more preferably at least 50. The PEGs may be branched PEGs. The number of branches may be 3, 4, 5, 6, 7, and 8. At least two of the termini in the branched PEGs may be covalently conjugated with a hydrophilic group and a hybrophobic group, respectively. In preferred embodiments, the amphiphilic molecules of the invention, wherein the hydrophilic group and hydrophobic group are connected by a linker, include the amphiphilic molecule with a hydrophobic group of polycyclic aromatic hydrocarbon derivative, preferably pyrene. In other preferred embodiments, the amphiphilic molecules of the invention, wherein the hydrophilic group and hydrophobic group are connected by a linker, include the amphiphilic molecule with a hydrophobic group of a cycloalkyne, preferably a cyclooctyne derivative, even more preferably a benzo-fused cyclooctyne derivative, and even more preferably dibenzocyclooctyne (DBCO). In various embodiments, the hydrophilic groups and hydrophobic groups of the amphiphilic molecules of the invention as described by any of the embodiments above, are not connected by a linker. In preferred embodiments, the amphiphilic moleculed of the invention, wherein the hydrophilic group and hydrophobic group are not connected by a linker, include the amphiphilic molecule with a hydrophobic group of aliphatic amine, preferably a fatty amine derivative, more preferably an octadecylamine derivative, and even more preferably dioctadecylamine (DODA). In preferred embodiments, the amphiphilic molecules of the invention, wherein the hydrophilic group and hydrophobic group are not connected by a linker, include the amphiphilic molecules with a hydrophobic group of a surface adhesive protein, preferably bovine serum albumin (BSA).

According to any of the above embodiments, the amphiphilic molecules may be considered as matrix molecules having a hydrophobic group and a hydrophilic group. Said hydrophobic group may comprise any one of: a polycyclic aromatic hydrocarbon derivative, preferably pyrene; a cycloalkyne, preferably a cyclooctyne derivative, more preferably a benzo-fused cyclooctyne derivative; a surface adhesive protein, preferably bovine serum albumin. The hydrophobic group and the hydrophilic groups may or may not be connected by a linker, wherein said linker preferably is a polymer, more preferably wherein said linker is poly(ethylene glycole) (PEG).

In various embodiments, the amphiphilic molecules may be considered as matrix molecules wherein the hydrophilic group of the amphiphilic compounds used for the (bio)functionalization of the graphene layer of the invention does not comprise a protein affinity capturing moiety. Said matrix molecules are able to form self-assembled monolayer. Said amphiphilic molecules include lipids, preferably 1,2-diacyl-sn-glycero-3-phosphocholine, more preferably any one of 1,2-dimyristoyl-sn-glycero-3-phosphocholine (DMPC), 1,2-dipalmitoyl-sn-glycero-3-phosphocholine (DPPC), 2-dilauroyl-sn-glycero-3-phosphocholine (DLPC), 1,2-dioleoyl-sn-glycero-3-phosphocholine (DOPC), 1,2-distearoyl-sn-glycero-3-phosphocholine (DSPC), and 1-myristoyl-2-palmityl-sn-glycero-3-phosphocholine (MPPC), 1-myristoyl-2-stearyl-sn-glycero-3-phosphocholine (MSPC), 1-palmityl-2-myristoyl-sn-glycero-3-phosphocholine (PMPC), 1-palmityl-2-stearyl-sn-glycero-3-phosphocholine (PSPC), 1-stearyl-2-myristoyl-sn-glycero-3-phosphocholine (SMPC), 1-stearyl-2-palmityl-sn-glycero-3-phosphocholine (SPPC), 1-myristoyl-2-oleoyl-sn-glycero-3-phosphocholine (MOPC), 1-palmityl-2-oleoyl-sn-glycero-3-phosphocholine (POPC), 1-stearyl-2-oleoyl-sn-glycero-3-phosphocholine (SOPC); and/or 1,2-diacyl-sn-glycero-3-phosphoethanolamines, more preferably, 1,2-dimyristoyl-sn-glycero-3-phosphoserine (DMPS), 1,2-dipalmitoyl-sn-glycero-3-phosphoserine (DPPS), 1,2-dioleoyl-sn-glycero-3-phosphoserine (DOPS), 1,2-distearoyl-sn-glycero-3-phosphoserine (DSPS), 1-palmityl-2-oleoyl-sn-glycero-3-phosphoserine (POPS), and 1-stearoyl-2-oleoyl-sn-glycero-3-phosphoserine (SOPS). The matrix lipid molecules are exemplified in Examples 2, 3 and 4. These matrix lipid molecules can be neutral in charge or negatively charge or a mixture thereof. In various embodiments, the molar ratio of negatively charged lipids in the mixture of negatively charged and neutral lipids is in the range 0 to 20%, 1%, 2%, 3%, 4% preferably 5%. In various embodiments, the molar ratio of negatively charged lipids in the mixture of negatively charged and neutral lipids is 10%, 15%, or 20%. In preferred embodiments, said molecules are 1,2-dioleoyl-sn-glycero-3-phosphocholine (DOPC), or 1,2-dioleoyl-sn-glycero-3-phosphoserine (DOPS), or a binary mixture thereof. In such embodiments, the molar ratio of DOPC/DOPS is 95:5.

In various embodiments, the graphene layer is functionalized with a mixture of matrix molecules comprising an affinity capture moiety according to any of the embodiments described herein and matrix molecules without an affinity capturing moiety according to any of the embodiments disclosed herein. In various embodiments, the molar ratio of matrix molecules with an affinity capturing moiety and without an affinity capturing moiety for the (bio)functionalization of graphene may be in the range between 0.001 to 0.999, preferably 0.01 to 0.99. In other preferred embodiments, the molar ratio may be 50:50, as shown in Example 12 for trisNTA-PEG-Pyrene/mPEG-Pyrene. In other preferred embodiments, the molar ratio may be 95:5, as shown in Example5 for DOPC/tris-NTA DODA.

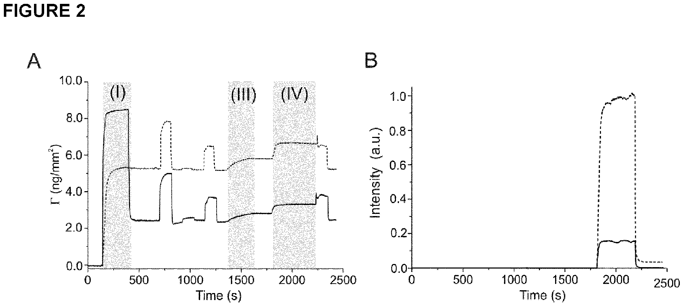

In various embodiments, the invention provides methods of synthesizing amphiphilic compounds for the biofunctionalization of graphene, as demonstrated in Examples 10, 11, 13 and 14. In various embodiments, the amphiphilic compounds comprising an affinity capturing moiety in the hydrophilic group are trisNTA-PEG-DBCO, trisNTA-PEG-Pyrene, trisNTA-DODA, BSA-HaloTag Ligand conjugate, and BSA-Biotin conjugate. In preferred embodiments, the amphiphilic compounds not comprising an affinity capturing moiety in the hydrophilic group may be 1,2-dioleoyl-sn-glycero-3-phosphocholine (DOPC), or 1,2-dioleoyl-sn-glycero-3-phosphoserine (DOPS), or a binary mixture thereof. In various embodiments, the amphiphilic molecules according to any of the embodiments described herein may be synthesized as monolayers or bilayers. The synthesis of a mono- and/or bilayer can be detected by mass change. Preferably, the mass change detected with the bilayer is twice as much as the mass change detected with a monolayer. In an exemplary embodiment, formation of a bilayer with a characteristic mass change of 5.0 ng/mm² and the exact half mass change characterizing the formation of a monolayer given by 2.5 ng/mm² is shown in Figure 2A. In various exemplary embodiments, the invention provides methods of synthesizing DOPC liposomes or binary mixtures of DOPS and DOPC liposomes for the biofunctionalization of graphene. The anionic DOPS is preferably included in the mixture at 5% by volume. Preferably, biofunctionalization with DOPS ensures perpendicular deposition of negatively charged biomolecules on the graphene layer.

In a third aspect, the invention provides a device having a support layer and a [bio]functionalized graphene layer on top of the support layer obtainable according to any of the embodiments of the first aspect of the present invention, and including any of the embodiments of the second aspect of the present invention.

As disclosed herein, the device comprises a graphene layer coated on top of a support layer. In various embodiments of the present invention, the support layer of the device provides a suitably strong foundation for the layer of bio-functionalized graphene. In preferred embodiments, the support layer of the present invention is a silica support layer. Preferably, the silica support layer comprises any one of a silica-type material, a UV-curable plastic material, glass, silicon, and/or a thermal plastic material, preferably wherein the support layer comprises a silica-type substrate, more preferably silica glass, which may be, e.g., 1x1 cm² silica-coated transducers or glass microscopy coverslips. In preferred embodiments, silica as used herein is in the form of silica-coated transducers. In a further preferred embodiment, the silica-coated transducers are for the purpose of reflectance interference spectroscopy and total internal reflection fluorescence spectroscopy detection (Rlfs-TIRFs). In other preferred embodiments, silica substrates as used herein are glass microscopy coverslips.

In various embodiments, the methods and GIET devices of the invention have a graphene layer, which is functionalized with the amphiphilic compounds of the invention disclosed herein. Preferably, the methods and GIET devices of the invention have a graphene layer on a silica support layer that is functionalized with any of the amphiphilic molecules according to any of the embodiments described herein above in the context of the second aspect of the invention, including in particular DOPC liposomes or binary mixtures of DOPS and DOPC liposomes, or trisNTA-PEG-DBCO, trisNTA-PEG-Pyrene, trisNTA-DODA, BSA-HaloTag Ligand conjugate and/or BSA-Biotin conjugate. Each alternative represents a separate embodiment of the invention.

In other embodiments, the invention provides a kit comprising a device manufactured according to the embodiments described above. The device can be used as a graphene induced energy transfer device.

In a fourth aspect, the invention provides methods of calibration of graphene induced energy transfer (GIET) versus distance making use of the devices disclosed herein. Graphene's gapless energy band structure and linear dispersion relation near the corners of the Brillouin zone result in a frequency independent light absorption, governed solely by the fine structure constant, α ≈ 1/137. As a result, this only one-atom thick material absorbs as much as πα ≈ 2.3% of light, over the visible and near-infrared spectral regions. As a consequence, graphene behaves as an extraordinary energy sink and a unique acceptor system. Fluorescent dyes placed close to graphene are strongly quenched and their displacement from graphene can restore fluorescence. It has been demonstrated experimentally that the energy transfer from a molecule (a single dipole) to graphene ('2D array of dipoles') strongly depends on the distance d between both and scales proportional to *d⁻⁴.* Whereas the distance dependence is well understood, reports vary with respect to the *d₀* value, which states the distance of 50% quenching efficiency. The method of calibration provided herein determines the sensitive graphene induced energy transfer region using both fluorescence intensity measurements (e.g. total internal reflection mode) and fluorescence lifetime measurements. In preferred embodiments, the method utilizes the device manufactured as described elsewhere herein with a graphene layer coated on top of a support layer. In preferred embodiments, the support layer of the present invention is a silica support layer. Preferably, the silica support layer comprises any one of a silica-type material, a UV-curable plastic material, glass, silicon, and/or a thermal plastic material, preferably wherein the support layer comprises a silica-type substrate, more preferably silica glass, which may be 1x1 cm² silica-coated transducers or glass microscopy coverslips. In preferred embodiments, silica as used herein is in the form of silica-coated transducers. In further preferred embodiments, the silica-coated transducers are for the purpose of reflectance interference spectroscopy and total internal reflection fluorescence spectroscopy detection (Rlfs-TIRFs). In other preferred embodiments, silica substrates as used herein are glass microscopy coverslips. In other embodiments, the invention provides a device, wherein the graphene layer is (bio)functionalized with the amphiphilic compounds synthesized according to the invention. Preferably, the device provided by the present invention comprises a graphene layer on a silica support layer that is (bio)functionalized with any of the amphiphilic molecules according to any of the embodiments described herein above in the context of the second aspect of the invention, including any of DOPC liposomes or binary mixtures of DOPS and DOPC liposomes, or trisNTA-PEG-DBCO, trisNTA-PEG-Pyrene, trisNTA-DODA, BSA-HaloTag Ligand conjugate and/or BSA-Biotin conjugate. Each alternative represents a separate embodiment of the invention.

In various embodiments of the invention, biomolecules are used as nanorulers for the methods of calibration of graphene induced energy transfer (GIET) versus distance. In preferred embodiments, single stranded DNA (ssDNA) is used as scaffold for nanorulers. Preferably, nanorulers of ssDNA comprise an 'anchor' strand and a 'probe' strand, wherein the two strands are hybridized. In preferred embodiments, the 'anchor' strand comprises at least common 20 mer sequence at the 5'-end and cholesterol modification at the 3'-end. Preferably, the anchor strand is attached to the non-covalently modified graphene layer coated on top of the silica support layer via the cholesterol modification at the 3'-end. In other preferred embodiments, the 'probe' strand comprises at least a common 20 mer sequence complementary to the 'anchor strand'. Preferably, ssDNA used as nanorulers comprise a fluorescence labeling. In preferred embodiments, the fluorescence label is on the 'probe' strand. As used herein, the term 'fluorescent labeling' refers generally to molecules which emit light at an emission wavelength following excitation of the label with light of an excitation wavelength. The emissions of fluorescent labels cover the detectable ultra violet light, the whole spectrum of visible light, and the detectable near infra-red regime. Preferably, the spectral regime of emission is in the range of 300 nm to 2500 nm. More preferably, 350 nm to 2000 nm. Most preferably, 400 nm to 800 nm. Examples of specific materials which can be used as fluorescent labels include organic dyes such as cyanine dyes, xanthene dyes, rhodamine dyes, fluorescein and fluorescein derivatives, and chelates of ions of rare-earth metals. 'Fluorescent labeling' as used herein further means an organic molecule having a fluorescent moiety and a linking moiety, such that the linking moiety allows the fluorescent moiety to be covalently attached to a target functionality. Some considerations associated with the construction and use of fluorescent labels include the stability of the bond between the fluorescent moiety and the linking moiety, the affect that the linking moiety has on fluorescent characteristics before and after attachment to a target functionality, the stability of the bond between the fluorescent moiety and the target functionality, i.e., the strength of the linking moiety after attachment, the reactivity of the linking functionality with the target functionality, and the like. When multiple fluorescent labels are required, each having distinct fluorescent characteristics, such as non-overlapping emission peaks, selection of labels for a particular application can become very difficult, often necessitating tradeoffs between the desired characteristics of the available fluorescent labels.

For the GIET measurements, the analyte or the interaction partner of the analyte may be fluorescently labeled. Fluorophores for labeling nucleotides can be organic dyes as here described. Fluorophores used for labeling the proteins can be organic dye molecules and fluorescent proteins (FPs). The FPs comprise green fluorescence proteins (e.g. GFP, mNeon), blue fluorescent proteins (e.g. BFP, CFP), red fluorescent proteins (e.g. mCherry, TagRFP), ultra red fluorescent proteins (URFPs).

In preferred embodiments, the fluorescence labeling is 6-carboxyfluorescein (FAM). In preferred embodiments, any number of ssDNA with sizes between 20 mer to 50 mer nucleotides can be used. For example, four ssDNA are used including 20 mer, 25 mer, 35 mer and 50 mer nucleotides as illustrated in Figure 1A. The method of calibration of GIET versus distance may involve obtaining fluorescence intensities of the biomolecule. In an exemplary embodiment, an ssDNA probe strand on both the graphene layer given by (I_{GIET}) and on silica alone given by (I₀). The ratio of the fluorescence intensities on graphene and silica allow for the quantification of distance (d)-dependent GIET efficiency. According to various embodiments of the invention, the sensitive GIET region is between 1-50 nm, preferably 1-40 nm, more preferably between 1-30 nm, and even more preferably between 1 nm and 25 nm. In other preferred embodiments, the GIET sensitivity within the sensitive GIET region may be between 1.0 to 3.0 nm, 1.5 to 3.0 nm, 2.0 to 3.0 nm, or 2.5 to 3.0 nm. The GIET sensitivity within the sensitive GIET region may in particular be less than about 1.7 nm, 1.6 nm, 1.5 nm, 1.4 nm, 1.3 nm or 1.2 nm. These results are depicted in the plot shown in Figure 1B. According to the validated intensity-based GIET curve, the I_{GIET}/ I₀ ratio at 30 nm is 140-fold of that at 1 nm, 9.2-fold of that at 5 nm, 2.2-fold of that at 10 nm, and 1.1-fold of that at 20 nm. The detection sensitivity at 1 nm is 15 times higher than that at 5 nm, 64 times higher than that at 10 nm and 127 times higher than that at 20 nm by using intensity ratios.

According to the fifth aspect of the invention, means and methods disclosed herein for biofunctionalized graphene on a support layer can be used as device in particular for DNA hybridization assays that are involved in clinical PCR (polymerase chain reaction) tests of target genomic sequences. In various embodiments of the invention as represented in Example 7, 8 and 9, the fluorescence quenching and/or de-quenching by DNA hybridization allow using GIET for DNA or RNA hybridization assays. The DNA or RNA hybridization assays may be carried out by fluorescence intensity measurements (e.g. total internal reflection mode) or fluorescence lifetime measurements. The method utilizes the device manufactured as described elsewhere herein with a graphene layer coated on top of a support layer. In preferred embodiments, the support layer of the present invention is a silica support layer. Preferably, the silica support layer comprises any one of a silica-type material, a UV-curable plastic material, glass, silicon, and/or a thermal plastic material, preferably wherein the support layer comprises a silica-type substrate, more preferably silica glass, which may be 1x1 cm² silica-coated transducers or glass microscopy coverslips. In other preferred embodiments, silica substrates as used herein are glass microscopy coverslips. In other embodiments, the invention provides a device, wherein the graphene layer is (bio)functionalized with the amphiphilic compounds synthesized according to the invention. The methods of fluorescence quenching and de-quenching by GIET are particularly useful to substitute molecular beacon probes or other quenching-based probes employed in polymerase chain reaction (PCR) detections. For instance, it can be used for real time PCR quantification or multiplex PCR assays for detecting the target genomic sequences.

According to a sixth aspect of the invention, provided herein are methods of determining the structure and/or dynamics of a biomolecule. As used herein, the term 'Biomolecule' includes any type of biomolecule for which (quantitative) structure, function, and/or activity determination may be desired, including, but not limited to, peptides, proteins, nucleic acids, sugars, mono- and polysaccharides, lipids, lipoproteins, whole cells, and the like, or a variety of enzymes, organic and inorganic chemicals, other sensitive biopolymers including DNA and RNA, and complex systems including whole or fragments of plant, animal and microbial cells. The term 'protein" as used herein indicates a polypeptide with a particular secondary and tertiary structure that can interact with another molecule, and in particular with other biomolecules including other proteins, DNA, RNA, lipids, metabolites, hormones, chemokines, and/or small molecules. The term 'polypeptide" as used herein indicates an organic linear, circular, or branched polymer composed of two or more amino acid monomers and/or analogs thereof. The term 'polypeptide' includes amino acid polymers of any length including full-length proteins and peptides, as well as analogs and fragments thereof. A polypeptide of three or more amino acids is also called a protein oligomer, peptide, or oligopeptide. In particular, the terms 'peptide' and 'oligopeptide' usually indicate a polypeptide with less than 100 amino acid monomers. In particular, in a protein, the polypeptide provides the primary structure of the protein, wherein the term 'primary structure' of a protein refers to the sequence of amino acids in the polypeptide chain covalently linked to form the polypeptide polymer. A protein 'sequence' indicates the order of the amino acids that form the primary structure. Covalent bonds between amino acids within the primary structure can include peptide bonds or disulfide bonds, and additional bonds identifiable by a skilled person. Polypeptides in the sense of the present disclosure are usually composed of a linear chain of alpha-amino acid residues covalently linked by peptide bond or a synthetic covalent linkage. The two ends of the linear polypeptide chain encompassing the terminal residues and the adjacent segment are referred to as the carboxyl terminus (C-terminus) and the amino terminus (N-terminus) based on the nature of the free group on each extremity. Unless otherwise indicated, counting of residues in a polypeptide is performed from the N-terminal end (NH2-group), which is the end where the amino group is not involved in a peptide bond to the C-terminal end (-COOH group) which is the end where a COOH group is not involved in a peptide bond. In some instances where the proteins are synthetic proteins in at least a portion of the polymer two or more amino acid monomers and/or analogs thereof are joined through chemically-mediated condensation of an organic acid (-COOH) and an amine (-NH2) to form an amide bond or a 'peptide' bond.

As used herein, the term 'amino acid' refers to organic compounds composed of amine and carboxylic acid functional groups, along with a side-chain specific to each amino acid. In particular, amino acid refers to organic compounds composed of amine (-NH2) and carboxylic acid (-COOH), and a side-chain specific to each amino acid connected to an alpha carbon. Different amino acids have different side chains and have distinctive characteristics, such as charge, polarity, aromaticity, reduction potential, hydrophobicity, and pKa. Amino acids can be covalently linked to form a polymer through peptide bonds by reactions between the amine group of a first amino acid and the carboxylic acid group of a second amino acid. Amino acid in the sense of the disclosure refers to any of the twenty naturally occurring amino acids, non-natural amino acids, and includes both D an L optical isomers.

The term 'ligand' as used herein refers to a moiety that is capable of covalently or otherwise chemically binding a compound of interest.

The term 'lipid' as used herein comprises mono-, di- and triglycerides of fatty acids, fatty acid sucrose and propyleneglycol esters and the like, and waxes, phospholipids and sugar lipids, as well as mixtures of the foregoing.

The term 'carbohydrate' as used herein includes polysaccharides (e.g., starches and dextrins) and sugars (e.g. sucrose, lactose, maltose, glucose, and fructose) that are metabolized for energy when hydrolyzed.

The term 'nucleic acids' as used herein may include any polymer or oligomer of pyrimidine and purine bases, preferably cytosine, thymine, and uracil, and adenine and guanine, respectively. See Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

As used herein, an 'aptamer' refers to a nucleic acid that has a specific binding affinity for a target molecule. It is recognized that affinity interactions are a matter of degree; however, in this context, the 'specific binding affinity' of an aptamer for its target means that the aptamer binds to its target generally with a much higher degree of affinity than it binds to other components in a test sample. An 'aptamer' is a set of copies of one type or species of nucleic acid molecule that has a particular nucleotide sequence. An aptamer can include any suitable number of nucleotides, including any number of chemically modified nucleotides. 'Aptamers' refers to more than one such set of molecules. Different aptamers can have either the same or different numbers of nucleotides. Aptamers can be DNA or RNA or chemically modified nucleic acids and can be single stranded, double stranded, or contain double stranded regions, and can include higher ordered structures. An aptamer can also be a photoaptamer, where a photoreactive or chemically reactive functional group is included in the aptamer to allow it to be covalently linked to its corresponding target. Any of the aptamer methods disclosed herein can include the use of two or more aptamers that specifically bind the same target molecule. As further described below, an aptamer may include a tag. If an aptamer includes a tag, all copies of the aptamer need not have the same tag. Moreover, if different aptamers each include a tag, these different aptamers can have either the same tag or a different tag.

The immobilization and capture of the biomolecule is characteristic for the method of determining the structure and/or dynamics of biomolecules. In various embodiments, the method of immobilizing the biomolecule to the graphene layer requires an affinity capturing moiety linked to the hydrophilic group of any of the amphiphilic molecules of the invention disclosed herein. In various embodiments, the method of immobilizing the biomolecule to the graphene layer requires a functional moiety linked to the biomolecule. In various embodiments, the method of immobilizing the biomolecule to the graphene layer requires affinity capturing moiety linked to the hydrophilic group of any of the amphiphilic molecules of the invention and a functional moiety linked to the biomolecule. The term 'functional moiety' as used herein refers to a chemical group or molecule which has a certain biological, chemical, therapeutic and/or diagnostic function *ex vivo* or *in vivo.* Functional moieties for use in the present invention are described elsewhere herein. In the present invention, the method of determining the structure and/or dynamics of biomolecules requires the hydrophilic group of the amphiphilic compounds of the invention comprising an affinity capturing moiety binding to the functional moiety of the biomolecule for immobilization of the biomolecule on the surface of the functionalized graphene layer of the device.

In various embodiments, the 'affinity capturing moiety' is an enzymatic protein capturing ligand preferably a haloalkane derivative, more preferably a chloroalkane derivative. In more preferred embodiments, the affinity capturing moiety is a HaloTag Ligand and the functional moiety is a HaloTag. HaloTag as used herein is a self-labeling protein tag. It is a 297 residue peptide (33 kDa) derived from a bacterial enzyme, designed to covalently bind to a synthetic ligand, the HaloTag ligand, which can be fused to a protein of interest. The HaloTag may be chosen from a number of available ligands known to the skilled person in the art. In further preferred embodiments, enzymatic protein capturing ligand is a 6-cholrohexanol derivative, preferably chlorohexane-OEG2-amine.

As described elsewhere herein, the 'affinity capturing moiety' may be a chelating agent. As used herein, the term 'chelating agent' is a chemical compound that coordinates with a metal to form a chelate. It comprises molecules containing two or more electron donor atoms that can form coordinate bonds to a single metal ion. The term 'chelating agent' is understood to include the chelating agent as well as salts thereof. In preferred embodiments, the chelating agent as used herein is a carboxylic acid-based chelator, more preferably a di- or triacetic acid-based chelator. In the preferred embodiments, the chelating agent as used herein may be a nitrilotriacetic acid (NTA) based agent. Accordingly, the functional moiety on the biomolecule is the poly histidine tag. The term 'poly histidine tag', as used herein refers to a linear sequence of histidine residues allowing for affinity based immobilization of the peptide and/or protein. A poly-His tag may comprise any of 6, 7, 8, 9,10, 11, 12, 13, or 14 consecutive Histidine residues.

In an exemplary embodiment, a hexahistidine-tagged functional moiety on a biomolecule is used to immobilize it on a device of the invention via the affinity capturing moiety, tris-NTA, on the hydrophilic group of the amphiphilic molecule on the functionalized graphene layer. In such exemplary embodiments, the biomolecule is a monomeric enhanced green fluorescence protein (H6GFP) as shown in Example 12 and Figure 6, phase III and IV.

In another exemplary embodiment, a HaloTag functional moiety functional moiety on a biomolecule is used to immobilize it on a device of the invention via the affinity capturing moiety, HaloTag ligand, on the hydrophilic group of the amphiphilic molecule on the functionalized graphene layer. In such exemplary embodiments, the biomolecule is antiGFP DARPin as shown in Example 15.

For determining the structure and/or dynamics of the biomolecules, after immobilizing the biomolecule to the functionalized graphene layer of the device, the next step is to determine the axial dimension of the biomolecule via fluorescence quenching. As used herein, the term 'fluorescence quenching' means reduction in fluorescence due to photochemical destruction of the fluorescent substance by irradiation with the excitation light. As used herein, the term 'fluorescent labeling' refers generally to molecules which emit light at an emission wavelength following excitation of the label with light of an excitation wavelength. The emissions of fluorescent labels cover the detectable ultra violet light, the whole spectrum of visible light, and the detectable near infra-red regime. Preferably, the spectral regime of emission is in the range of 300 nm to 2500 nm. More preferably, 350 nm to 2000 nm. Most preferably, 400 nm to 800 nm. Examples of specific materials which can be used as fluorescent labels include organic dyes such as cyanine dyes, xanthene dyes, rhodamine dyes, fluorescein and fluorescein derivatives, and chelates of ions of rare-earth metals. 'Fluorescent labeling' as used herein further means an organic molecule having a fluorescent moiety and a linking moiety, such that the linking moiety allows the fluorescent moiety to be covalently attached to a target functionality. Some considerations associated with the construction and use of fluorescent labels include the stability of the bond between the fluorescent moiety and the linking moiety, the affect that the linking moiety has on fluorescent characteristics before and after attachment to a target functionality, the stability of the bond between the fluorescent moiety and the target functionality, i.e., the strength of the linking moiety after attachment, the reactivity of the linking functionality with the target functionality, and the like. When multiple fluorescent labels are required, each having distinct fluorescent characteristics, such as non-overlapping emission bands, selection of labels for a particular application can become very difficult, often necessitating tradeoffs between the desired characteristics of the available fluorescent labels.

For the GIET measurements, the analyte or the interaction partner of the analyte may be fluorescently labeled. Fluorophores for labeling nucleotides can be organic dyes as here described. Fluorophores used for labeling the proteins can be organic dye molecules and fluorescent proteins (FPs). The FPs comprise green fluorescence proteins (e.g. GFP, mNeon), blue fluorescent proteins (e.g. BFP, CFP), red fluorescent proteins (e.g. mCherry, TagRFP), ultra red fluorescent proteins (URFPs).

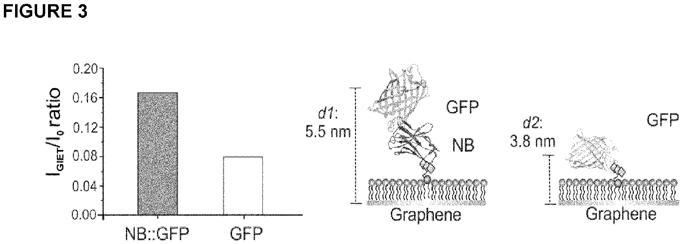

The term 'axial dimension' as used herein is defined as being a nominal lengthwise dimension (nm) of the biomolecule (s). In an exemplary embodiment, axial dimension of H6-NB::GFP complex is determined as shown in Example 5 and Figures 2A, 2B and 3.

In yet other embodiments, the axial conformational change of a biomolecule can be determined. As used herein, the term 'conformational change' refers to the alteration of the molecular structure of a substance. It is intended that the term encompass the alteration of the structure of a single molecule or molecular aggregate. The term 'conformational change', as used herein, when used in reference to proteins, means at least one change in the structure of the protein, a change in the shape of the protein or a change in the arrangement of parts of the protein. The parts of the protein can be, for example, atoms that change relative location due to rotation about one or more chemical bonds occurring in the molecular structure between the atoms. The parts can also be regions of secondary, tertiary or quaternary structure. The parts of the protein can further be domains of a macromolecule, such as those commonly known in the relevant art.

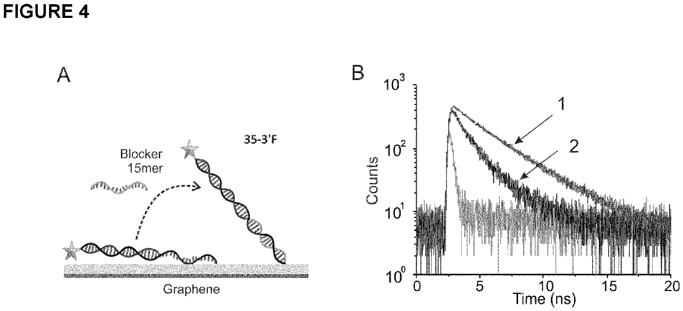

In an exemplary embodiment the conformational change of ssDNA is determined as shown in Example 6 and Figures 4A and 4B. In preferred embodiments, the axial conformational change of larger than 10 nm is quantified.

According to the methods and compositions of the invention, it has been revealed that GIET has an extended sensitive distance up to 50 nm with nanometric sensitivity, in comparison with FRET'S 10 nm. According to the invention, GIET requires only single fluorescence labeling on the biomolecule of interest, unlike FRET measurements that need double labeling of both donor and acceptor on proteins. As a consequence, corrections of the labeling degree and spectroscopic bleeding through necessary for FRET measurements are avoided. According to the methods of the present invention, the high transparency of graphene allows lifetime determination by GIET being completed very quickly within a few seconds, while MIET requires typically 10 minutes to collect enough photons for accurate determination of fluorescence lifetime.

The invention also shows that due to the high transparency of graphene to visible light, fluorescence intensity-based GIET can be done in the format of total internal reflection fluorescence detection. Thus, highly sensitive, single molecule GIET can be realized according to the invention to obtain the structural fluctuations of individual biomolecules that are hidden by assemble measurements. According to the aspects of the invention, biofunctionalization of graphene by trisNTA-PEG-Pyrene or trisNTA-PEG-DBCO is reversible upon alcohol washing. Moreover, His-tagged proteins are reversibly bound to graphene via trisNTA.

In the seventh aspect of the invention, the methods, compounds, and devices disclosed herein can be used in screening, analytical and/or diagnostic applications. The means and methods of the invention, including the novel amphiphilic compounds trisNTA-PEG-DBCO, trIsNTA-PEG-Pyrene, and BSA-HaloTag Ligand conjugate for biofunctionalization of graphene, can be used in detecting the structure and activity of a large spectrum of biomolecules, exemplified herein by DNA and protein. The means and methods disclosed herein for biofunctionalized graphene on a support layer can be used as device in particular for DNA hybridization assays that are involved in clinical PCR (polymerase chain reaction) tests of target genomic sequences. Moreover, the means and methods can be used as device for structural dynamic assays of pharmaceutically important transmembrane receptors, cytokine receptors, GPCRs, or soluble His-tagged proteins. These GIET devices will be the core component of diagonal kits for a broad application in scientific research and drug screening (cf. the quantitative PCR instruments, and the commercialized Biacore^{®} chips). The kit can report drug-induced structural change of protein receptors as index of its effectiveness. Automatic, large scale screening of target genes and drug candidates can be realized by integrating GIET devices within fluidic systems, sampling systems, etc.

### EXAMPLES

Embodiments of means and methods of the present invention are illustrated in the following examples. These examples are provided for illustrative purposes only and are not considered to be limiting the scope of the invention to any extent.

### Example 1 Formation of graphene monolayer on silica substrates

Silica-type substrates were used for formation of graphene monolayer on top. They include 1×1 cm² silica-coated transducers for reflectance interference spectroscopy and total internal reflection fluorescence spectroscopy detection (Rlfs-TIRFs), and glass microscopy coverslips. The Rlfs-TIRFs transducers were cleaned in fresh Piranha solution (one part 30% H₂O₂ and two parts concentrated H₂SO₄ - caution, highly corrosive). Glass microscopy coverslips were cleaned by plasma cleaner (Femto plasma system, Diener electronic GmbH/Germany) under vacuum obtained from ambient air. For coating the substrates with graphene monolayer, graphene monolayer together with a thin film of protection polymer (Easy Transfer Monolayer^{®}, Graphenea Inc, Spain) was cut into 5x5 mm² pieces. The pieces were slowly emerged into MiliQ water to float on top of water. The polymer-protected graphene monolayer was fished out by clean glass coverslip or silica Rlfs-TIRFs transducer from below. This led to face-to-face contact of graphene with the substrates. The obtained substrates were left at room temperature for drying 30 min, followed by heating in a 150°C oven for 2 h. The hot substrates were then taken out from oven and immediately stored under vacuum for 24 h for cooling down. Afterwards, the substrates were incubated in acetone for 1 h, sequentially in isopropanol for another 1 h, to remove the protection polymer film on graphene. By blow-drying with N₂, the graphene monolayer coated substrates were ready for use.

### Example 2 Preparation of liposomes for noncovalent functionalization of graphene

For preparation of DOPC liposome, 2.5 µmol DOPC dissolved in chloroform were mixed in a 50 mL round bottom flask. For preparation of liposome containing DOPC:DOPS (95:5 molar ratio), 2.4 µmol DOPC and 0.12 µmol DOPS dissolved in chloroform were mixed in a 50 ml round bottom flask. Similarly, lipid mixture containing 2.4 µmol DOPC and 0.125 µmol tris-nitrilotriacetic acid-dioctadecylamine (trisNTA-DODA) was prepared the same way as for obtaining liposomes containing DOPC:trisNTA-DODA (95:5 molar ratio). For all the abovementioned lipid solutions, the chloroform was evaporated by a rotary evaporator, respectively. The dried lipids were re-suspended in 10 mL HBS buffer (20 mM HEPEs, 150 mM NaCl, pH 7.5) by vortexing 30 min. The lipid solution was then probe-sonicated 3 times for 5 min in ice-water batch. After sonication, the solution were centrifuged at 20000 rpm for 15 min. The supernatant is liposome solution containing 250 µM lipid or lipid mixture.

### Example 3 Calibration of distance-dependent graphene induced energy transfer (GIET) by intensity measurement

Distance dependency of GIET was calibrated on graphene monolayer-coated silica substrates which were noncovalently modified by lipid monolayer. Four single strand DNA (ssDNA) with 20 mer, 25 mer, 35 mer and 50 mer nucleotides were designed as the scaffold of nanorulers (Figure 1A). These ssDNAs share common 20 mer sequence at the 5'-end and cholesterol modification at the 3'-end as 'anchor' strands. To ensure perpendicular deposition of oligonucleotides to lipid layer, 5 % negatively charged DOPS was added into the matrix lipid DOPC, i.e. DOPC:DOPS (95:5 molar ratio). 'Probe' strands have a common 20 mer ssDNA sequence complementary to the anchor strand. They are conjugated with 6-carboxyfluorescein (FAM) at either 3'- or 5'-end, respectively, for fluorescence read-out. The hybridization of the probe and anchor strands are denoted as 'anchor strand-3'F or 5'F'. For the 35 mer and 50 mer anchor strands, additional complementary 15 mer and 30 mer ssDNA were used as blockers, respectively, to obtain the fully length double strand DNA nanorulers. All DNA sequences are listed in Table 1.

**Table 1 DNA strands used for calibration***

| DNA Strands | Sequence and modifications (5'-3') |
|---|---|
| SEQ ID NO: 1- 20mer anchor: | GATGAATGGTGGGTGAGAGG-TEG-Cholesterol |
| SEQ ID NO: 2 - 25 mer anchor: | GATGAATGGTGGGTGAGAGGTGAGG-Cholesterol |
| SEQ ID NO: 3 - 35 mer anchor: | GATGAATGGTGGGTGAGAGGTGAGGAGTA AGAGGA-Cholesterol |
| SEQ ID NO: 4 - 50 mer anchor: | GATGAATGGTGGGTGAGAGGTGAGGAGTAAGA GGATGTGTTAGAGGGATG-Cholesterol |
| SEQ ID NO: 5 - 3'-FAM probe: | CCTCTCACCCACCATTCATC-FAM |
| SEQ ID NO: 6 - 5'-FAM probe: | FAM-CCTCTCACCCACCATTCATC |
| SEQ ID NO: 7 - 15 mer blocker: | TCCTCTTACTCCTCA |
| SEQ ID NO: 8 - 30 mer blocker: | CATCCCTCTAACACATCCTCTTACTCCTCA |

| | |
|---|---|
| *custom synthesized by IDT DNA technology | |

A home-built flow-through system with simultaneous reflectance interference spectroscopy and total internal reflection fluorescence spectroscopy (Rlfs-TIRFs) detection was used for surface sensitive detection (*see* Gavutis et al. 2006, Nature Protocols, Vol. 1, 2091). To prepare solid supported lipid layers, liposomes containing 250 µM DOPC/DOPS (95:5 molar ratio) prepared in Example 2 was injected into the flow chamber mounted on top of the solid substrates. Alternatively, solution-assisted lipid deposition was used for forming lipid monolayer on graphene. In this approach, liposomes containing 250 µM DOPC/DOPS (95:5 molar ratio) in HBS was dissolved in a mixture solution of HBS:EtOH (90:10 v/v). The mixture was injected into the flow chamber of Rlfs-TIRFs setup. After formation of lipid layers on solid support, cholesterol-modified ssDNA was injected for immobilizing the anchor strand on surface. Sequentially, FAM-labeled ssDNA was injected as probe strand for hybridization with anchor strand. All DNA concentrations were 1 µM. Running buffer for DNA hybridization was HBS buffer containing Mg ions (HBS-Mg, 20 mM HEPES, 150 mM NaCl and 5 mM MgCl₂, pH 7.5). The Rlfs-TIRFs signals of forming lipid monolayer on graphene, immobilization of ssDNA anchor strands, and hybridization with probe strands on lipid layers were monitored in real-time, respectively. The obtained fluorescence intensities of probe strand on graphene (I_{GIET}) were compared to those on silica (I₀) with respect to the mass signals of immobilized DNA strands, respectively. By assuming a tilting angle α between DNA and graphene, we calculated the vertical distance d. Altogether, seven measured I_{GIET}/ I₀ ratios were plotted against d (Figure 1). The obtained correlation of I_{GIET}/ I₀ vs d matches the theoretical GIET curve with a globally fitted tilting angle of 43 ± 1°. According to the validated intensity-based GIET curve, the I_{GIET}/ I₀ ratio at 30 nm is 140-fold of that at 1 nm, 9.2-fold of that at 5 nm, 2.2-fold of that at 10 nm, and 1.1-fold of that at 20 nm. The detection sensitivity at 1 nm is 15 times higher than that at 5 nm, 64 times higher than that at 10 nm and 127 times higher than that at 20 nm by using intensity ratios.

### Example 4 Calibration of distance-dependent GIET by lifetime measurement

Alternatively, formation of lipid monolayer on graphene, immobilization of ssDNA anchor strands, and hybridization with probe strands on lipid layers were carried out in vitro on a graphene-coated glass microscopy coverslip. The coverslips were mounted in a microscopy chamber having 1 mL buffer volume. 800µL of DOPC/DOPS (molar ratio of 95:5) liposome solution prepared in Example 2 was added on top of coverslip and incubated for 30 min at room temperature. The coverslip was washed with excess HBS to remove free liposome in solution. Afterwards, the coverslip was incubated with 1 µM of different ssDNA anchor strands for 15 min, respectively, then washed 10 times by HBS-Mg buffer. 100 nM of probe strands was added to solution and incubated for 15 min, followed by 10 times HBS washing. Fluorescence quenching by graphene was quantified by time-correlated single photon counting (TCSPC) using confocal laser scanning microscope (Olympus FluoView 1000) equipped with a PicoQuant system (PicoHarp 300). All TCSPC traces were fit with mono-exponential decay functions. Ratios of lifetimes on graphene to those on glass (τ_{GIET}/τ₀) were grouped according to 3'-or 5'-labeling of the probe strand, respectively. The obtained plots of τ_{GIET}/τ₀ from seven DNA nanorulers were plotted versus the vertical distance d in the same way as described in Example 3 (Figure 1B). Results of both intensity and lifetime measurements show a sensitive GIET region within 1-30 nm. Significant difference were detected for nanorulers between 35-5'F and 35-3'F. The results confirm that GIET is highly sensitive to distance change of 1.2 nm.

### Example 5 Determination of protein axial dimension using intensity-based GIET

Site-specific capturing His-tagged proteins on graphene monolayer-coated silica-transducer was carried out in home-built Rlfs-TIRFs setup. Liposomes containing 250 µM DOPC/trisNTA-DODA (95:5 molar ratio) was injected into the flow chamber mounted on top of the solid substrates. Alternatively, solution-assisted lipid deposition was used for formation of lipid monolayer on graphene. In this approach, ethanol was added to DOPC/trisNTA-DODA in HBS to get a final mixture of HBS:EtOH (90:10 volume ratio). Formation of DOPC/tris-NTA DODA (95:5 molar ratio) lipid bilayer on silica transducer showed a characteristic mass change of 5.0 ng/mm. Strikingly, the mass signal of lipid deposition on graphene was 2.5 ng/mm². The exact half mass signal indicates formation of densely packed trisNTA-DODA/DOPC lipid monolayer on graphene (Figure 2A). After conditioning trisNTA-DODA/DOPC lipid monolayer on graphene by loading with Ni²⁺ ions, 1 µM hexahistidine tagged anti-GFP nanobody (H6-NB) was injected, followed by injection of 100 nM monomeric enhanced green fluorescence protein (GFP) (Figure 2B). The association constant of mEGFP and NB interaction, and quantitative removal of H6-NB::GFP complex via imidazole verified the high affinity capturing of His-tagged protein with intact function on graphene. By comparing the fluorescence signal (I_{GIET}) to that on silica substrate (I₀) that were normalized with immobilized protein amount, an I_{GIET}/I₀ ratio of 16.4% was obtained by normalizing to the immobilized protein amount. Direct immobilization of H6-tagged GFP on graphene resulted to I_{GIET}/I₀ ratio of 7.6 %. Using the calibrated GIET to distance curve, the I_{GIET}/I₀ ratio of 7.6 % for immobilized H6-tagged GFP corresponds to 3.8 nm of GFP fluorophore above graphene. The obtained I_{GIET}/I₀ ratio of 16.4 % for NB::GFP complex indicates that the GFP fluorophore in the complex is 5.5 nm above graphene (Figure 3).

### Example 6 Quantifying the axial conformational change of DNA using lifetime -based GIET

Graphene monolayer-coated coverslips were mounted in a microscopy chamber having 1 mL buffer volume. 800µL of DOPC/DOPS (molar ratio of 95:5) liposome solution prepared in Example 2 was added on top of coverslip and incubated for 30 min at room temperature. The coverslip was rinsed with excess HBS to remove free liposome from the surface. Afterwards, 1 µM 35 mer ssDNA anchor strand was anchored on graphene coated with lipid monolayer (DNA sequences are listed in Table 1). By hybridizing with 100 nM 20 mer 3' FAM probe strand in solution, a lifetime τ_{GIET} of 0.24 ns was detected by TCSPC using confocal laser scanning microscopy. Comparing to τ₀ of 3.0 ns on glass, the obtained τ_{GIET}/τ₀ ratio of 8 % indicates a distance of 4.4 nm to graphene. This suggests that the hybridized probe-anchor DNA strand lays on the top of lipid monolayer due to flexible, unpaired 15 mer gap. Upon addition of 200 nM 15 mer ssDNA blocker strand in solution, a lifetime of 1.5 ns was observed (Figure 4B). According to the calibration curve, the τ_{GIET}/τ₀ ratio of 49 ± 3% corresponds to a distance of 10.9 ± 0.2 above graphene. The results depict that the 35 mer ssDNA anchor strand hybridized with probe sticks to the lipid monolayer at the rest state. Binding of 15 mer blocker makes the flexible gap rigid by additional hybridization to dsDNA and extending to its full length (Figure 4). The results confirm that GIET is feasible to measure molecular distance changes larger than 10 nm.

### Example 7 DNA hybridization assays by intensity-based GIET

A home-built flow-through system with simultaneous reflectance interference spectroscopy and total internal reflection fluorescence spectroscopy (Rlfs-TIRFs) detection was used for surface sensitive detection (*see* Gavutis et al. 2006, Nature Protocols, Vol. 1, 2091). To prepare solid supported lipid layers, liposomes containing 250 µM DOPC/DOPS (95:5 molar ratio) prepared in Example 2 was injected into the flow chamber mounted on top of the solid substrates. Alternatively, solution-assisted lipid deposition was used for forming lipid monolayer on graphene. After formation of lipid layers on solid support, 1 µM cholesterol-modified ssDNA was injected for immobilizing the anchor strand on surface: GATGAATGGTGGGTGAGAGGTGAGG-Cholesterol (the 25 mer anchor) given by SEQ ID NO: 2. Sequentially, 1 µM FAM-labeled ssDNA, CCTCTCACCCACCATTCATC-FAM given by SEQ ID NO: 5 was injected as probe strands for hybridization with anchor strand. Running buffer for DNA hybridization was HBS buffer containing Mg ions (HBS-Mg, 20 mM HEPES, 150 mM NaCl and 5 mM MgCl₂, pH 7.5). The Rlfs-TIRFs signals of forming lipid monolayer on graphene, immobilization of ssDNA anchor strands, and hybridization with probe strands on lipid layers were monitored in real-time, respectively. The obtained fluorescence intensities of probe strand on graphene (I_{GIET}) were compared to those on silica (I₀) with respect to the mass signals of immobilized DNA strands. The I_{GIET}/ I₀ ratios of 23.2 ± 1.4 % reported a significant quenching of fluorescence intensity for the probe ssDNA. This result confirmed significant quenching of fluorescently labeled probe DNA strand upon hybridization with DNA strand immobilized on graphene-supported lipid monolayer.

### Example 8 DNA hybridization assays by intensity-based GIET with an unlabeled DNA strand

The same procedure of forming lipid layers on solid supports was implemented as Example 7. Next, 1 µM cholesterol-modified ssDNA was injected for immobilizing on surface: GATGAATGGTGGGTGAGAGGTGAGGAGTAAGAGGATGTGTTAGAGGGATG-Cholesterol (the 50 mer anchor) given by SEQ ID NO: 4. Sequentially, 1 µM probe strands FAM-labeled ssDNA, CCTCTCACCCACCATTCATC-FAM given by SEQ ID NO: 5 or FAM-CCTCTCACCCACCATTCATC given by SEQ ID NO: 6 was injected together with 1µM unlabeled ssDNA strand CATCCCTCTAACACATCCTCTTACTCCTCA given by SEQ ID NO: 8 on solid supports for hybridization with the anchor strand. Running buffer for DNA hybridization was HBS buffer containing Mg ions (HBS-Mg, 20 mM HEPES, 150 mM NaCl and 5 mM MgCl₂, pH 7.5). The Rlfs-TIRFs signals of forming lipid monolayer on graphene, immobilization of ssDNA anchor strands, and hybridization with probe strands on lipid layers were monitored in real-time, respectively. The obtained fluorescence intensities of probe strand on graphene (I_{GIET}) were compared to those on silica (I₀) with respect to the mass signals of immobilized DNA strands. The I_{GIET}/ I₀ ratios of 63.8 ± 1.9 % and 41.9 ± 0.3 % reported significant quenching of fluorescence intensities of the two probe ssDNAs, respectively. The results confirmed sensitive detection of DNA hybridization on graphene-supported lipid monolayer using fluorescently labeled DNA strands in combination with an unlabeled DNA strand.

### Example 9 DNA hybridization assays by lifetime-based GIET

Graphene monolayer-coated coverslips were mounted in a microscopy chamber having 1 mL buffer volume. 800µL of DOPC/DOPS (molar ratio of 95:5) liposome solution prepared in Example 2 was added on top of coverslip and incubated for 30 min at room temperature. The coverslip was rinsed with excess HBS to remove free liposome from the surface. Afterwards, 1 µM GATGAATGGTGGGTGAGAGGTGAGGAGTAAGAGGA-Cholesterol (the 35 mer ssDNA anchor strand) given by SEQ ID NO: 3 was anchored on graphene coated with lipid monolayer. By hybridizing with 100 nM CCTCTCACCCACCATTCATC-FAM (the 20 mer 3' FAM probe strand) given by SEQ ID NO: 5 in solution, a lifetime τ_{GIET} of 0.24 ns was detected by TCSPC using confocal laser scanning microscopy. Upon addition of 200 nM TCCTCTTACTCCTCA (the 15 mer ssDNA blocker strand) given by SEQ ID NO: 7 in solution, a lifetime τ_{GIET} of 1.5 ns was observed (Figure 4B). Altogether, around 6-fold increase of fluorescence lifetime was obtained by the two-step DNA hybridization. The results show that lifetime-based GIET can detect hybridization of fluorescently labeled DNA strand on graphene-supported lipid monolayer in presence of an unlabeled DNA strand in solution.

### Example 10 Synthesis of trisNTA-PEG-DBCO

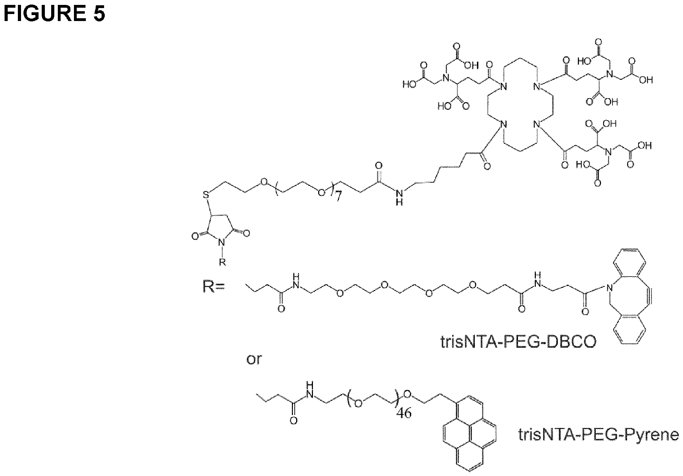

11 mg trisNTA-OEG7-disulfide (Mw 2978 Da, self-made) was dissolved in 200 µL HBS buffer. 2.2 mg tris(2-carboxyethyl)phosphine hydrochloride (TCEP, Mw 286.6 Da, Sigma Aldrich, C4706) in 100 µL HBS was added to the trisNTA-OEG7-disulfide solution. The mixture were reacted for 15 min at pH 7.5 to obtain reduced thiol compound of trisNTA-OEG7-SH. The mixture was added to a 10 mL glass round flask that contains 300 µL N,N-dimethylformamide (DMF) solution of 5 mg dibenzocyclooctyne-PEG4-maleimide (DBCO-PEG4-Mal, Mw 674.7 Da, Sigma Aldrich, 760676). The reaction mixture was stirred by magnetic bar for 1 h. The crude product was diluted in 10 mL MiliQ water having 0.2% (v/v) trifluoroacetic acid (TFA). Crude product was further purified by C18 RP-HPLC with a gradient of 0-100 % acetonitrile in 0.2% TFA/water. The purified compound was lyophilized as white powder. Yield: 7.6 mg. MS (MALDI-TOF): 2187 [M+Na]⁺. The obtained compound was named as trisNTA-PEG-DBCO (Figure 5), stored as 1 mg aliquots in glass vials at -20°C.

### Example 11 Synthesis of trisNTA-PEG-Pyrene

5 mg trisNTA-OEG7-disulfide (Mw 2978 Da, self-made) was dissolved in 200 µL HBS buffer. 1.0 mg tris(2-carboxyethyl)phosphine hydrochloride (TCEP, Mw 286.6 Da, Sigma Aldrich, C4706) in 100 µL HBS was added to the trisNTA-OEG7-disulfide solution. The mixture were reacted for 15 min at pH 7.5 to obtain reduced thiol compound of trisNTA-OEG7-SH. The mixture was added to a 10 mL glass round flask that contains 300 µL N,N-dimethylformamide (DMF) solution of 5 mg Pyrene-PEG2000-maleimide (Pyrene-PEG2k-Mal, Average Mw 2000 Da, Creative PEGworks Inc, PBL-9062). The reaction mixture was stirred by magnetic bar for 1 h. The crude product was diluted in 10 mL MiliQ water having 0.2% (v/v) trifluoroacetic acid (TFA) and purified by C18 RP-HPLC with a gradient of 0-100 % acetonitrile in 0.2% TFA/water. The purified compound was lyophilized. Yield: 5.8 mg white powder. MS (MALDI-TOF): 3512 [M+Na]+ with multiple ±44 m/z peaks. The obtained compound was named as trisNTA-PEG-Pyrene (Figure 5), which was stored as 1 mg aliquots in glass vials at -20 °C.

### Example 12 Functionalization of graphene by pi-pi interaction with trisNTA-PEG-Pyrene

1 mg trisNTA-PEG-Pyrene and 1 mg mPEG-Pyrene (Pyrene-PEG2k-OMe, Average Mw 2000 Da, Creative PEGworks Inc, PCN-102) was dissolved into water:ethanol (90:10 v/v). 50 µM trisNTA-PEG-Pyrene/mPEG-Pyrene (50:50 molar ratio) in water:ethanol (90:10 v/v) was injected into the flow chamber of the home-built Rlfs-TIRFs setup. Formation of trisNTA-PEG-Pyrene/mPEG-Pyrene layer on graphene was characterized by the mass signal of 2.0 ng/mm2 (Figure 6, phase I). The stable immobilization of trisNTA-PEG-Pyrene/mPEG-Pyrene monolayer confirms strong pi-pi interaction of pyrene on graphene. After conditioning of the trisNTA-PEG-Pyrene/mPEG-Pyrene layer on graphene by Ni2+ loading, 1 µM hexahistidine-tagged monomeric enhanced green fluorescence protein (H6-GFP) was injected (Figure 6, phase III and IV). The stable immobilization of H6-GFP and quantitative removal of H6-NB::GFP complex via imidazole verified the specific capturing of His-tagged protein on trisNTA-PEG-Pyrene/mPEG-Pyrene-modified graphene.

### Example 13 Synthesis of BSA-HaloTag Ligand conjugate

75 mg bovine serum albumin (BSA Fraction V, Carl-Roth) was dissolved in 4 mL HBS. 96 mg N-(3-Dimethylaminopropyl)-N'-ethylcarbodiimide hydrochloride (EDC, Sigma Aldrich 03449) was dissolved in 1 mL HBS. 3.9 mg chlorohexane-OEG2-amine (HaloTag Ligand, HTL, self-synthesized) was dissolved in 200 µL ethanol. The three solutions were mixed up in a 10 mL round flask and magnetically stirred for 4 h at 4 °C. The reaction mixture was dialyzed in HBS buffer for 48 h at 4 °C using a membrane with cut-off size of 14 kDa. UV-vis absorption at 280 nm indicated the obtained solution contains 1.5 mM BSA-HTL conjugate. The solution was frozen by liquid nitrogen and stored in -80°C.

### Example 14 Synthesis of BSA-Biotin conjugate

BSA-Biotin was synthesized in the sample way as described in Example 13, except that 4.1 mg biotin (Sigma Aldrich B4501) was used instead of 3.9 mg HaloTag Ligand. A final solution of 1.8 mM BSA-Biotin was obtained and stored in -80°C.

### Example 15 Functionalization of graphene by amphiphilic molecules comprising surface adhesive protein

20 µL of BSA-HTL solution of Example 9 was applied to graphene monolayer-coated silica transducer, which then face-to-face contacted with another graphene-coated transducer. Afterwards, the transducers were separated and washed by HBS for 3 times. Transducers were incubated with 10 µM HaloTag-fused antiGFP DARPin, which is a specific binder to GFP. The obtained transducers were washed by HBS buffer for 3 times and stored in HBS buffer at 4 °C before use. GFP binding assays were carried out in the home-built Rlfs-TIRFs setup. 500 nM monomeric enhance green fluorescence protein (GFP) was injected in the flow chamber of Rlfs-TIRFs setup. Immobilization of GFP on graphene was characterized by the mass signal of 1.5 ng/mm2 (Figure 7, gray bar). In contrast, there was negligible GFP immobilization on BSA treated graphene monolayer coated on transducer. The results confirmed that amphiphilic BSA-HTL formed stable functionalization layer on graphene. Thus, Halotag-fused antiGFP DARPin was immobilized on graphene via covalent coupling of HTL and HaloTag. GFP was then specifically captured from solution to the amphiphilic molecule functionalized graphene surface.

## Claims

1. A method of manufacturing a device having a support layer and a biofunctionalized graphene layer on top of the support layer, **characterized in that** the method comprises the steps:
(a) transferring a layer of graphene to a support layer to form a support layer with a graphene layer on top;
(b) applying an amphiphilic coating to the graphene layer of step (a) to form a non-covalent surface coating on the graphene layer,
wherein the non-covalent surface coating comprises amphiphilic molecules having a hydrophobic group and a hydrophilic group connected by a polymeric linker, wherein the hydrophobic group is bound to the graphene layer and comprises any one of: a polycyclic aromatic hydrocarbon derivative, a cycloalkyne, a surface adhesive protein, and/or an aliphatic amine;
and
(c) obtaining a device having a support layer and a biofunctionalized graphene layer on top of the support layer.

2. The method of claim 1, wherein the amphiphilic coating is up to 5 nm on top of the graphene layer.

3. The method of claim 1 or 2, wherein:
(i) the hydrophilic group comprises an affinity capturing moiety, and/or
(ii) the hydrophobic group comprises any one of: pyrene; a cyclooctyne derivative, preferably a benzo-fused cyclooctyne derivative; bovine serum albumin; and/or a fatty amine derivative, preferably an octadecylamine derivative.

4. The method of claim 3, wherein the affinity capturing moiety is a target analyte capturing moiety and wherein the target analyte is a biomolecule including any one of a peptide or protein, an aptamer, a nucleic acid, a lipid and a carbohydrate, preferably wherein the target analyte is a proteinogenic molecule or a nucleic acid molecule, more preferably protein, DNA or RNA.

5. The method of claim 4, wherein the affinity capturing moiety is
(a) a protein capturing ligand, preferably wherein the protein capturing ligand is a haloalkane derivative, more preferably a chloroalkane derivative; or
(b) a nucleic acid capturing ligand, preferably wherein the nucleic acid capturing ligand is complementary nucleic acid sequence, avidin or streptavidin, or a binding protein; or
(c) a chelating agent, preferably a carboxylic acid-based chelator, more preferably a di- or triacetic acid-based chelator, preferably wherein the chelating agent is a multivalent chelator.

6. The method of any one of claims 2 to 5, wherein the linker is poly(ethylene glycole) (PEG).

7. The method of claim 1 or 2, wherein the amphiphilic coating comprises amphiphilic molecules without an affinity capturing moiety.

8. The method of any one of claims 1 to 7, further comprising a step of immobilizing a target analyte to the non-covalently modified surface of the graphene layer of step (c).

9. The method of claim 8, wherein the target analyte is immobilized to the non-covalent surface of the graphene layer via a functional moiety targeting or linked to the one or more amphiphilic molecules, preferably wherein the functional moiety is targeting or linked to the hydrophilic group of the one or more amphiphilic molecules.

10. The method of any one of claims 1 to 9, wherein the support layer comprises any one of a silica-type material, a UV-curable plastic material, glass, silicon, and/or a thermal plastic material, preferably wherein the support layer comprises a silica-type substrate, more preferably silica glass.

11. A graphene induced energy transfer (GIET) device having a support layer and a [bio]functionalized graphene layer on top of the support layer manufactured by the method of any one of claims 1 to 10.

12. A method of determining the structure and/or dynamics of a target analyte comprising immobilizing said target analyte to the GIET device of claim 11 and determining the distance dependent GIET efficiency, preferably via fluorescence quenching.

13. The method of claim 12, which is for determining the axial dimension and/or the axial conformational change of a target analyte.

14. The GIET device of claim 11, or the method of claim 12 or 13, wherein the sensitive GIET region is about 1-50 nm, preferably 3-40 nm, more preferably 5-30 nm.

15. Use of the GIET device of claim 11, in screening, analytical and/or diagnostic applications.

## Patentansprüche

1. Verfahren zur Herstellung einer Vorrichtung mit einer Trägerschicht und einer biofunktionalisierten Graphenschicht auf der Trägerschicht, **dadurch gekennzeichnet, dass** das Verfahren die Schritte umfasst:
(a) Übertragen einer Graphenschicht auf eine Trägerschicht, um eine Trägerschicht mit einer darauf befindlichen Graphenschicht zu bilden;
(b) Aufbringen einer amphiphilen Beschichtung auf die Graphenschicht von Schritt (a), um eine nicht-kovalente Oberflächenbeschichtung auf der Graphenschicht zu bilden,
wobei die nicht-kovalente Oberflächenbeschichtung amphiphile Moleküle mit einer hydrophoben Gruppe und einer hydrophilen Gruppe, welche durch einen polymeren Linker verbunden sind, umfasst, wobei die hydrophobe Gruppe an die Graphenschicht gebunden ist und eines der folgenden umfasst: ein polyzyklisches aromatisches Kohlenwasserstoffderivat, ein Cycloalkin, ein Oberflächenhaftprotein, und/oder ein aliphatisches Amin;
und
(c) Erhalten einer Vorrichtung mit einer Trägerschicht und einer biofunktionalisierten Graphenschicht auf der Trägerschicht.

2. Das Verfahren nach Anspruch 1, wobei die amphiphile Beschichtung bis zu 5 nm auf der Graphenschicht beträgt.

3. Das Verfahren nach Anspruch 1 oder 2, wobei:
(i) die hydrophile Gruppe eine Affinitätsfängereinheit umfasst, und/oder
(ii) die hydrophobe Gruppe eine der folgenden umfasst: Pyren; ein Cyclooctin-Derivat, vorzugsweise ein benzokondensiertes Cyclooctin-Derivat; Rinderserumalbumin; und/oder ein Fettamin-Derivat, vorzugsweise ein Octadecylamin-Derivat.

4. Das Verfahren nach Anspruch 3, wobei die Affinitätsfängereinheit eine Zielanalytenfängereinheit ist und wobei der Zielanalyt ein Biomolekül ist, einschließlich eines Peptids oder Proteins, eines Aptamers, einer Nukleinsäure, eines Lipids, und einem Kohlenhydrat,
wobei der Zielanalyt vorzugsweise ein proteinogenes Molekül oder ein Nukleinsäuremolekül ist, besonders bevorzugt Protein, DNA oder RNA.

5. Das Verfahren nach Anspruch 4, wobei die Affinitätsfängereinheit
(a) ein Protein-einfangender Ligand ist, wobei der Protein-einfangende Ligand vorzugsweise ein Halogenalkanderivat, besonders bevorzugt ein Chloralkanderivat ist; oder
(b) ein Nukleinsäure-einfangender Ligand ist, wobei der Nukleinsäure-einfangende Ligand vorzugsweise eine komplementäre Nukleinsäuresequenz, Avidin oder Streptavidin, oder ein Bindeprotein ist; oder
(c) ein Chelatbildner ist, vorzugsweise ein Chelatbildner auf Carbonsäurebasis, noch bevorzugter einen Chelatbildner auf Di- oder Triessigsäurebasis, wobei der Chelatbildner vorzugsweise ein mehrwertiger Chelatbildner ist.

6. Das Verfahren nach einem der Ansprüche 2 bis 5, wobei der Linker Poly(ethylenglykol) (PEG) ist.

7. Das Verfahren nach Anspruch 1 oder 2, wobei die amphiphile Beschichtung amphiphile Moleküle ohne eine Affinitätsfängereinheit umfasst.

8. Das Verfahren nach einem der Ansprüche 1 bis 7, welches ferner einen Schritt der Immobilisierung eines Zielanalyten an die nicht-kovalent modifizierte Oberfläche der Graphenschicht von Schritt (c) umfasst.

9. Das Verfahren nach Anspruch 8, wobei der Zielanalyt über eine funktionelle Einheit an die nicht-kovalente Oberfläche der Graphenschicht immobilisiert ist, welche auf das eine oder die mehreren amphiphilen Moleküle abzielt oder mit ihnen verbunden ist, wobei die funktionelle Einheit vorzugsweise auf die hydrophile Gruppe des einen oder der mehreren amphiphilen Moleküle abzielt oder mit ihnen verbunden ist.

10. Das Verfahren nach einem der Ansprüche 1 bis 9, wobei die Trägerschicht ein Material vom Siliziumdioxid-Typ, ein UV-härtbares Kunststoffmaterial, Glas, Silizium und/oder ein thermisches Kunststoffmaterial umfasst, wobei die Trägerschicht vorzugsweise ein Substrat vom Siliziumdioxid-Typ, insbesondere Siliziumdioxidglas, umfasst.

11. Graphen-induzierte Energieübertragungs (GIET)-Vorrichtung mit einer Trägerschicht und einer [bio]funktionalisierten Graphenschicht auf der Trägerschicht, hergestellt gemäß dem Verfahren nach einem der Ansprüche 1 bis 10.

12. Verfahren zur Bestimmung der Struktur und/oder Dynamik eines Zielanalyten, umfassend das Immobilisieren des Zielanalyten an die GIET-Vorrichtung nach Anspruch 11 und das Bestimmen der abstandsabhängigen GIET-Effizienz, vorzugsweise durch Fluoreszenzlöschung.

13. Das Verfahren nach Anspruch 12, zur Bestimmung der axialen Dimension und/oder der axialen Konformationsänderung eines Zielanalyten.

14. Die GIET-Vorrichtung nach Anspruch 11, oder das Verfahren nach Anspruch 12 oder 13, wobei der empfindliche GIET-Bereich etwa 1-50 nm, vorzugsweise 3-40 nm, noch bevorzugter 5-30 nm, beträgt.

15. Verwendung der GIET-Vorrichtung nach Anspruch 11 in Screening-, Analyse- und/oder Diagnoseanwendungen.

## Revendications

1. Méthode de fabrication d'un dispositif comportant une couche de support et une couche de graphène biofonctionnalisée sur la couche de support, **caractérisée par le fait que** la méthode comprend les étapes suivantes:
(a) transfert d'une couche de graphène sur une couche de support pour former une couche de support recouverte d'une couche de graphène;
(b) appliquer un revêtement amphiphile sur la couche de graphène de l'étape (a) pour former un revêtement de surface non covalent sur la couche de graphène, dans lequel le revêtement de surface non covalent comprend des molécules amphiphiles ayant un groupe hydrophobe et un groupe hydrophile reliés par un lien polymère, dans lequel le groupe hydrophobe est lié à la couche de graphène et comprend l'un des éléments suivants: un dérivé d'hydrocarbure aromatique polycyclique, un cycloalcyne, une protéine adhésive de surface, et/ou une amine aliphatique;
et
(c) l'obtention d'un dispositif comportant une couche de support et une couche de graphène biofonctionnalisée sur la couche de support.

2. La méthode de la revendication 1, dans laquelle le revêtement amphiphile se trouve jusqu'à 5 nm au-dessus de la couche de graphène.

3. La méthode de la revendication 1 ou 2, dans laquelle:
(i) le groupe hydrophile comprend une fraction capturant l'affinité, et/ou
(ii) le groupe hydrophobe comprend l'un des éléments suivants: pyrène; dérivé de cyclooctyne, de préférence un dérivé de cyclooctyne fusionné au benzo; albumine de sérum bovin; et/ou dérivé d'amine grasse, de préférence un dérivé d'octadécylamine.

4. La méthode de la revendication 3, dans laquelle la fraction de capture d'affinité est une fraction de capture d'analyte cible et dans laquelle l'analyte cible est une biomolécule comprenant un peptide ou une protéine, un aptamère, un acide nucléique, un lipide et un hydrate de carbone, de préférence dans laquelle l'analyte cible est une molécule protéinogène ou une molécule d'acide nucléique, plus préférentiellement une protéine, de l'ADN ou de l'ARN.

5. La méthode de la revendication 4, dans laquelle la fraction capturant l'affinité est
(a) un ligand de capture des protéines, de préférence dans lequel le ligand de capture des protéines est un dérivé d'haloalcane, plus préférentiellement un dérivé de chloroalcane; ou
(b) un ligand de capture d'acide nucléique, de préférence dans lequel le ligand de capture d'acide nucléique est une séquence d'acide nucléique complémentaire, de l'avidine ou de la streptavidine, ou une protéine de liaison; ou
(c) un agent chélateur, de préférence un chélateur à base d'acide carboxylique, plus préférentiellement un chélateur à base d'acide di- ou triacétique, de préférence dans lequel l'agent chélateur est un chélateur multivalent.

6. La méthode de l'une des revendications 2 à 5, dans laquelle l'élément de liaison est un poly(éthylène glycole) (PEG).

7. La méthode de la revendication 1 ou 2, dans laquelle le revêtement amphiphile comprend des molécules amphiphiles sans fraction de capture d'affinité.

8. La méthode de l'une des revendications 1 à 7, comprenant en outre une étape d'immobilisation d'un analyte cible sur la surface modifiée de manière non covalente de la couche de graphène de l'étape (c).

9. La méthode de la revendication 8, dans laquelle l'analyte cible est immobilisé sur la surface non covalente de la couche de graphène par l'intermédiaire d'une fraction fonctionnelle ciblant ou liée à une ou plusieurs molécules amphiphiles, de préférence dans laquelle la fraction fonctionnelle cible ou est liée au groupe hydrophile d'une ou plusieurs molécules amphiphiles.

10. La méthode de l'une des revendications 1 à 9, dans lequel la couche de support comprend un matériau de type silice, un matériau plastique durcissant aux UV, du verre, du silicium et/ou un matériau plastique thermique, de préférence dans lequel la couche de support comprend un substrat de type silice, plus préférentiellement du verre de silice.

11. Dispositif de transfert d'énergie induit par le graphène (GIET) comportant une couche de support et une couche de graphène [bio]fonctionnalisé sur la couche de support fabriquée selon la méthode de l'une des revendications 1 à 10.

12. Méthode de détermination de la structure et/ou de la dynamique d'un analyte cible comprenant l'immobilisation dudit analyte cible sur le dispositif GIET de la revendication 11 et la détermination de l'efficacité GIET en fonction de la distance, de préférence via l'extinction de la fluorescence.

13. La méthode de la revendication 12, qui consiste à déterminer la dimension axiale et/ou le changement de conformation axiale d'un analyte cible.

14. Le dispositif GIET de la revendication 11, ou la méthode de la revendication 12 ou 13, dans lequel la région GIET sensible est d'environ 1-50 nm, de préférence 3-40 nm, plus préférentiellement 5-30 nm.

15. Utilisation du dispositif GIET de la revendication 11 dans des applications de dépistage, d'analyse et/ou de diagnostic.
